# EUROPEAN PATENT SPECIFICATION

(11) **EP 2 288 345 B1**
(45) Date of publication and mention of the grant of the patent: **10.06.2015**
(21) Application number: 09733496.5
(22) Date of filing: 20.04.2009
(51) Int. Cl.: A61K 31/045, A61K 45/06, A61P 25/00

(54) **PSYCHO-PHARMACEUTICALS**
PSYCHOPHARMAZEUTIKA
PRODUITS PSYCHO-PHARMACEUTIQUES

(30) Priority: 18.04.2008 IE 20080298
(43) Date of publication of application: 02.03.2011
(73) Proprietor: University College Dublin National University Of Ireland, Dublin, Dublin 4 (IE)
(72) Inventor: REGAN, Ciaran, Dublin 8 (IE); CONBOY, Lisa, Dublin 18 (IE); GANNON, Shane, Clonbur County Galway (IE)
(74) Representative: MacLachlan & Donaldson
(86) International application number: PCT/IE2009/000021
(87) International publication number: WO 2009/128058

(56) References cited:
- GB-A- 1 415 228
- US-A- 4 201 725
- US-A- 4 223 041
- SCHAFFARZICK R W ET AL: "The anticonvulsant activity and toxicity of methylparafynol (dormison) and some other alcohols." SCIENCE (NEW YORK, N.Y.) 12 DEC 1952, vol. 116, no. 3024, 12 December 1952 (1952-12-12), pages 663-665, XP002538385 ISSN: 0036-8075
- GUPTA B D ET AL: "Emotion as a determinant of the effects of drugs and their combination on different components of behaviour in rats" NEUROPHARMACOLOGY, PERGAMON PRESS, OXFORD, GB, vol. 11, no. 1, 1 January 1972 (1972-01-01), pages 31-38, XP025490183 ISSN: 0028-3908 [retrieved on 1972-01-01]
- MAY P R A ET AL: "Dormison, a new type of hypnotic: its therapeutic use in psychiatric patients" AMERICAN JOURNAL OF PSYCHIATRY, AMERICAN PSYCHIATRIC ASSOCIATION, WASHINGTON, DC, US, vol. 109, no. 12, 1 June 1953 (1953-06-01), pages 881-888, XP009120553 ISSN: 0002-953X
- KENNEDY D G ET AL: "Methylpentynol (oblivon) in the treatment of epilepsy." SCIENCE (NEW YORK, N.Y.) 16 SEP 1955, vol. 122, no. 3168, 16 September 1955 (1955-09-16), page 515, XP002538386 ISSN: 0036-8075
- MARRIOTT A S ET AL: "Effects of centrally acting drugs on exploratory behaviour in rats." BRITISH JOURNAL OF PHARMACOLOGY AND CHEMOTHERAPY OCT 1965, vol. 25, no. 2, October 1965 (1965-10), pages 432-441, XP002538476 ISSN: 0366-0826
- GUPTA ET AL: "An examination of the effects of stimulant and depressant drugs on escape/avoidance conditioning in strains of rats selectively bred for emotionality/non-emotionality: Intertrial activity" 19690501, vol. 8, no. 3, 1 May 1969 (1969-05-01), pages 227-234, XP022252590
- GILLHESPY R O: "Methylpentynol carbamate in the management of insomnia and parkinsonism" BRITISH JOURNAL OF CLINICAL PRACTICE,, vol. 12, no. 3, 1 March 1958 (1958-03-01), pages 191-193, XP009120346 ISSN: 0007-0947
- HELD R: "Trial therapy with methylpentynol carbamate in ambulatory psychiatric treatment" SEMAINE DES HOPITAUX DE PARIS, EXPANSION SCIENTIFIQUE FRANCAISE, PARIS, FR, vol. 32, no. 23, 14 April 1956 (1956-04-14), pages 1326-1333, XP009120390 ISSN: 0037-1777
- BROUSSOLLE P ET AL: "Effects of methylpentynol carbamate in a severe psychosis. Therapeutic action and critical phenomena" JOURNAL DE MEDECINE DE LYON, SN, LYON, FR, vol. 41, no. 960, 1 January 1960 (1960-01-01), pages 123-127, XP009120391 ISSN: 1157-0385
- HOUSER M ET AL: "Utilization of methylpentynol carbamate in large doses in psychiatry" JOURNAL DE MEDECINE DE LYON,, vol. 38, no. 918, 1 January 1958 (1958-01-01), pages 309-317, XP009120547
- CIRILLI M ET AL: "On the association of psychotonic drugs and C.M.P. (methylparafynol carbamate) in psychiatric therapy" ANNALES MEDICO-PSYCHOLOGIQUES, FORTIN, PARIS, FR, vol. 119, no. 1, 1 February 1961 (1961-02-01), pages 285-299, XP009120395 ISSN: 0003-4487
- LECOQ R ET AL: "The course of alcoholic desintoxication and its application in some types of serious encephalopathies" ENCEPHALE, PARIS, FR, vol. 53, no. 1, 1 January 1964 (1964-01-01), pages 35-61, XP009120397 ISSN: 0013-7006
- LEHMANN A ET AL: "A study of the effect of psychotropic drugs on the processes of excitation and inhibition of the CNS by the audiogenic crisis test in the rat" PSYCHOPHARMACOLOGY, SPRINGER VERLAG, BERLIN, DE, vol. 3, no. 5, 1 January 1962 (1962-01-01), pages 331-343, XP009120399 ISSN: 0033-3158
- MARLEY E ET AL: "Clinical aspects of susceptibility to methylpentynol." JOURNAL OF NEUROLOGY, NEUROSURGERY, AND PSYCHIATRY MAY 1958, vol. 21, no. 2, May 1958 (1958-05), pages 129-140, XP002538387 ISSN: 0022-3050
- DICKER S E ET AL: "The effect of methylpentynol in man." BRITISH JOURNAL OF PHARMACOLOGY AND CHEMOTHERAPY DEC 1957, vol. 12, no. 4, December 1957 (1957-12), pages 479-483, XP002538388 ISSN: 0366-0826
- J. J Hagan: "Predicting Drug Efficacy for Cognitive Deficits in Schizophrenia", Schizophrenia Bulletin, vol. 31, no. 4, 15 September 2005 (2005-09-15), pages 830-853, XP055063328, ISSN: 0586-7614, DOI: 10.1093/schbul/sbi058
- Philippe Pichat ET AL: "SSR180711, a Novel Selective [alpha]7 Nicotinic Receptor Partial Agonist: (II) Efficacy in Experimental Models Predictive of Activity Against Cognitive Symptoms of Schizophrenia", Neuropsychopharmacology, vol. 32, no. 1, 1 January 2007 (2007-01-01), pages 17-34, XP055063326, ISSN: 0893-133X, DOI: 10.1038/sj.npp.1301188

## Description

### TECHNICAL FIELD

The present invention relates to novel therapeutic uses of Meparfynol and its pharmaceutically acceptable salts. In particular, the present invention concerns the use of Methyl Pentynol (also known as Meparfynol) or a pharmaceutically acceptable salt thereof for the treatment and/or prevention of schizophrenia.

### BACKGROUND OF THE INVENTION

Psychosis is a symptom of severe mental illness. Although it is not exclusively linked to any particular psychological or physical state, it is particularly associated with schizophrenia, bipolar disorder (manic depression) and severe clinical depression. Psychosis is characterized by disorders in basic perceptual, cognitive, affective and judgmental processes. Individuals experiencing a psychotic episode may experience hallucinations (often auditory or visual hallucinations), hold paranoid or delusional beliefs, experience personality changes and exhibit disorganised thinking (thought disorder). This is sometimes accompanied by features such as a lack of insight into the unusual or bizarre nature of their behaviour, difficulties with social interaction and impairments in carrying out the activities of daily living.

Psychosis is not uncommon in cases of brain injury and may occur after drug use, particularly after drug overdose or chronic use. Certain compounds may be more likely to induce psychosis and some individuals may show greater sensitivity than others. The direct effects of hallucinogenic drugs are not usually classified as psychosis, as long as they abate when the drug is metabolised from the body. Chronic psychological stress is also known to precipitate psychotic states, however the exact mechanism is uncertain. Psychosis triggered by stress in the absence of any other mental illness is known as brief reactive psychosis. Psychosis is thus a descriptive term for a complex group of behaviours and experiences. Individuals with schizophrenia can have long periods without psychosis and those with bipolar disorder, or depression, can have mood symptoms without psychosis.

Hallucinations are defined as sensory perception in the absence of external stimuli. Psychotic hallucinations may occur in any of the five senses and can take on almost any form, which may include simple sensations (such as lights, colours, tastes, smells) to more meaningful experiences such as seeing and interacting with fully formed animals and people, hearing voices and complex tactile sensations. Auditory hallucination, particularly the experience of hearing voices, is a common and often prominent feature of psychosis. Hallucinated voices may talk about, or to the person, and may involve several speakers with distinct personas. Auditory hallucinations tend to be particularly distressing when they are derogatory, commanding or preoccupying.

Psychosis may involve delusional or paranoid beliefs, classified into primary and secondary types. Primary delusions are defined as arising out-of-the-blue and not being comprehensible in terms of normal mental processes, whereas secondary delusions may be understood as being influenced by the person's background or current situation, that is, represent a delusional interpretation of a "real" situation. Thought disorder describes an underlying disturbance to conscious thought and is classified largely by its effects on the content and form of speech and writing. Affected persons may also show pressure of speech (speaking incessantly and quickly), derailment or flight of ideas (switching topic mid-sentence or inappropriately), thought blocking, rhyming or punning. Psychotic episodes may vary in duration from individuals. In brief, reactive psychosis, the psychotic episode is commonly related directly to a specific stressful life event, so patients spontaneously recover normal functioning, usually within two weeks. In some rare cases, individuals may remain in a state of full blown psychosis for many years, or perhaps have attenuated psychotic symptoms (such as low intensity hallucinations) present at most times.

Psychoses exact a tremendous emotional and economic toll on the patients, their families, and society as a whole. Psychotic conditions, such as schizophrenia and related disorders (such as, for example, schizoaffective disorder), and including affective disorders (mood disorders) with psychotic symptoms (such as, for example, Bipolar Disorder) are complex and heterogeneous diseases of uncertain aetiology that afflict a large percentage of all populations world-wide.

Patients who suffer a brief psychotic episode may have many of the same symptoms as a person who is psychotic as a result of, for example, schizophrenia, and this fact has been used to support the notion that psychosis is primarily a breakdown in some specific biological system in the brain.

Schizophrenia is a major psychotic disorder affecting up to 1% of the population. Schizophrenia dramatically affects the health and well-being of individuals who suffer from this mental disorder, which is among the most severe and difficult to treat. Schizophrenia is a chronic, severe and disabling form of psychosis. It is found at similar prevalence in both sexes and is found throughout diverse cultures and geographic areas. The World Health Organization found schizophrenia to be the world's fourth leading cause of disability that accounts for 1.1% of the total DALYs (Disability Adjusted Life Years) and 2.8% of YLDs (years of life lived with disability). It was estimated that the economic cost of schizophrenia exceeded US$ 19 billion in 1991, more than the total cost of all cancers in the United States. Individuals with schizophrenia ("schizophrenics") can suffer from a myriad of symptoms and may require significant custodial care and continuous drug and/or behaviour therapy, leading to substantial social and economic costs, even in the absence of hospitalization or institutionalization. Schizophrenia affects approximately 2 million Americans. Early diagnosis and effective treatment of schizophrenia can improve prognosis and help reduce the costs associated with this illness.

Schizophrenia is a serious mental disorder whose cardinal features include impaired sensory processing, cognitive deficits and the presence of a withdrawn state. The illness usually develops from adolescence and age 30 and is characterized by one or more positive symptoms (such as, for example, delusions and hallucinations) and/or negative symptoms/cognitive impairment (such as, for example, blunted emotions and lack of interest) and/or disorganized symptoms (such as, for example, confused thinking and speech and/or disorganized behaviour and perception). Cognitive enhancers are much sought after remedies for schizophrenics' malady.

Schizophrenics have been demonstrated in many studies to have degraded abilities at tasks requiring short-term verbal working memory, rapidly associated cognitive "prediction" or "expectation", or ongoing attention/vigilance control. Schizophrenics who have auditory hallucinations (which describes the majority of afflicted individuals) also have a strongly correlated degradation in their speech reception abilities. Schizophrenics also have social and functional skill deficits, such as, for example, deficits and confusion in identifying the moods or reactions of others, in determining what for them is a socially correct course of action and in identifying the sources of current and past actions or events. Schizophrenia is a chronic disorder and most patients require constant treatment to alleviate or decrease the incidence of psychotic episodes. The causes of schizophrenia are largely unknown. Although it is believed to have a genetic component, environmental factors appear to influence the onset and severity of the disease.

From the discussion above, it is clear that the neuropsychological cognitive impairment in people with functional neuropsychiatric disorders, such as schizophrenia, is still an area of high unmet medical need with no effective drugs. Despite intensive research into the mechanism of the pathogenesis of schizophrenia and the development of new drugs for effective treatment of this disease, there also remains a significant interest in and need for additional or alternative therapies for treating, preventing and/or delaying the onset and/or development of schizophrenia. The moderate and inconsistent effect observed with some drugs leave space to identify more effective and safe treatments. In particular, there is a need for additional or alternative therapies which have efficacy in managing the core symptoms associated with schizophrenia. Current therapies can also cause unpleasant side-effects and lead to difficulties in maintaining patient compliance. In particular in some neuropsychological disease states, some interventions, often worsen cognitive functions. Thus, the need of new drugs that ameliorate cognition without worsening other side effects, is very high. In particular, there is a need for new therapeutic agents which can improve the quality of life for patients with symptoms associated with schizophrenia. SCHAFFARZICK R W ET AL: "The anticonvulsant activity and toxicity of methylparafynol (dormison) and some other alcohols.", SCIENCE (NEW YORK, N.Y.) 12 DEC 1952, vol.. 116, no. 3024, 12 December 1952, vol. 116, no. 3024, 12 December 1952 (1952-12-12), pages 663-665 and KENNEDY D G ET AL: "Methylpentynol (oblivon) in the treatment of epilepsy.", SCIENCE (NEW YORK, N.Y.) 16 SEP 1955, vol. 122, no. 3168, 16 SEPTEMBER 1955 (1955-09-16), page 515 disclose the use of methypent-1-yn-3-ol against epilepsy.

GUPTA B D ET AL: "Emotion as a determinant of the effects of drugs and their combination on different components of behaviour in rats", NEUROPHARMACOLOGY, PERGAMON PRESS, OXFORD, GB, vol. 11, no. 1, 1 January 1972 (1972-01-01), pages 31-38, MARRIOTT A S ET AL: "Effects of centrally acting drugs on exploratory behaviour in rats.", BRITISH JOURNAL OF PHARMACOLOGY AND CHEMOTHERAPY OCT 1965, vol. 25, no. 2, October 1965 (1965-10), pages 432-441 and GUPTA ET AL: "An examination of the effects of stimulant and depressant drugs on escape/avoidance conditioning in strains of rats selectively bred for emotionality/non-emotionality: Intertrial activity", 19690501, vol. 8, no. 3, 1 May 1969 (1969-05-01), pages 227-234 report compositions comprising 3-methypent-1-yn-3-ol for treating stress and anxiety.

MAY P R A ET AL: "Dormision, a new type of hypnotic: its therapeutic use in psychiatric patients", AMERICAN JOURNAL OF PSYCHIATRY, AMERICAN PSYCHIATRIC ASSOCIATION, WASHINGTON, DC, US, vol. 109, no. 12, 1 June 1953 (1953-06-01), pages 881-888 discloses the use of 3-methypent-1-yn-3-ol for treating sleep problems in schizophrenic patients.

GILLHESPY R O: Methylpentynol carbamate in the management of insomnia and parkinsonism", BRITISH JOURNAL OF CLINICAL PRACTICE, vol. 12, no. 3, 1 March 1958 (1958-03-01) pages 191-193 relates to the use of methylpentynol carbamate in the treatment of insomnia and Parkinson's disease.

HELD R: "Trial therapy with methylpentynol carbamate in ambulatory psychiatric treatment", SEMAINE DES HOPITAUX DE PARIS, EXPANSION SCIENTIFIQUE FRANCAISE, PARIS, FR, vol. 32, no. 23, 14 April 1956 (1956-04-14), pages 1326-1333, BROUSSOLLE P ET AL: "Effects of methylpentynol carbamate in a severe psychosis. Therapeutic action and critical phenomena", JOURNAL DE MEDECINE DE LYON, SN, LYON, FR, vol. 41, no. 960, 1 January 1960 (1960-01-01) pages 123-127, HOUSER M ET AL: "Utilization of methylpentynol carbamate in large doses in psychiatry", JOURNAL DE MEDECINE DE LYON, vol. 38, no. 918, 1 January 1958 (1958-01-01), pages 309-317 and CIRILLI M ET AL: "On the association of psychotonic drugs and C.M.P. (methylparafynol carbamate) in psychiatric therapy", ANNALES MEDICO PSYCHOLOGIQUES, FORTIN, PARIS, FR, vol. 119, no. 1, 1 February 1961 (1961-02-01) pages 285-299 disclose the use of methylpentynol carbamate against psychological disorders.

LECOQ R ET AL: "The course of alcoholic desintoxication and its application in some types of serious encephalopathies". ENCEPHALE, PARIS, FR, vol. 53, no. 1, 1 January 1964 (1964-01-01), pages 35-61 discloses methylpentynol carbamate in the treatment of alcohol related withdrawal and LEHMANN A ET AL: "A study of the effect of psychotropic drugs on the processes of excitation and inhibition of the CNS by the audiogenic crisis test in the rat", PSYCHOPHARMACOLOGY, SPRINGER VERLAG, BERLIN, DE, vol. 3, no. 5, 1 January 1962 (1962-01-01), pages 331-343 describe the use of the same drug in an audiogenic crisis.

US 4 223 041 A (PIGEROL CHARLES ET AL) 16 September 1980 (1980-09-16), US 4 201 725 A, (EPSE MADELEINE C [FR] ET AL) 6 May 1980 (1980-05-06) and GB 1 415 228 A (LABAZ) 26 November 1975 (1975-11-26) disclose compounds useful in the treatment of conditions such as anxiety, aggression, Parkinson's disease and epilepsy.

MARLEY E ET AL: "Clinical aspects of susceptibility to methylpentynol.", JOURNAL OF NEUROLOGY, NEUROSURGERY, AND PSYCHIATRY MAY 1958, vol. 21, no. 2, May 1958 (1958-05), pages 129-140 and DICKER S ET AL: "The effect of methylpentynol in a man.", BRITISH JOURNAL OF PHARMACOLOGY AND CHEMOTHERAPY DEC 1957, vol. 12, no. 4, December 1957 (1957-12) pages 479-483 disclose sedative effects of 3-methypent-1-yn-3-ol.

J. J HAGAN: "Predicting Drug Efficacy for Cognitive Deficits in Schizophrenia", Schizophrenia Bulletin, vol. 31, no. 4, 15 September 2005 (2005-09-15), pages 803-853 and PHILIPPE PICHAT ET AL: "SSR180711, a Novel Selective [alpha]7 Nicotinic Receptor Partial Agonist: (II) Efficacy in Experimental Models Predictive of Activity Against Cognitive Symptoms of Schizophrenia", Neuropsychopharmacology, vol. 32, no. 1, 1 January 2007 (2007-01-01), pages 17-34 disclose the use of clozapine, amisulpride, SSR181507 and SSR180711 in the treatment of schizophrenia.

### SUMMARY OF THE INVENTION

In accordance to this invention it has been found that Meparfynol (3-methyl-1-pentyn-3-ol), and its pharmaceutically acceptable salts, may be effective in treating schizophrenia.

Accordingly, the invention relates to Meparfynol, compositions including Meparfynol and uses thereof for treating schizophrenia as claimed in the appended claims.

To the Applicants knowledge, there is no example in the literature that Meparfynol could have a positive effect on sensory processing functions and/or cognitive functions. Given the lack of previous demonstration that Meparfynol has cognitive enhancing properties, as well as sensory processing enhancing properties, the present findings are unexpected. Meparfynol (3-methyl-1-pentyn-3-ol) and its pharmaceutically acceptable salts or mixtures may be administered orally. The advantages derived from the uses of the invention as above defined are many, and include the possibility to treat schizophrenia with a surprisingly favourable profile of safety. Other advantages are discussed and are made apparent in the following commentary.

In one embodiment described herein the compound modulates a cognitive function and/or a sensory processing function.

The disorder treatable by the compound of the invention has symptoms associated with a sensory processing impairment.

The invention also provides a pharmaceutical composition comprising a pharmaceutically acceptable carrier and, as an active ingredient, an effective amount of a compound of the invention for use in the treatment of schizophrenia.

In yet another aspect described herein, the composition may include one or more other agents selected from the group consisting of: (i) an NMDA/AMPA receptor modulator; (ii) a nootropic agent; (iii) an anti-depressive agent (eg SRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant. The treatment may comprise the simultaneous or sequential administration of Meparfynol and one or more additional agents selected from the group consisting of: (i) an NMDA/AMPA receptor modulator; (ii) a nootropic agent; (iii) an anti-depressive agent (eg SRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant.

Also described herein is a process for making the pharmaceutical composition described wherein the process comprises mixing Meparfynol and one or more additional agents selected from the group consisting of: (i) an NMDA/AMPA receptor modulator; (ii) a nootropic agent; (iii) an anti-depressive agent (eg SRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant.

The present invention in a further aspect provides use of Meparfynol or a pharmaceutically acceptable salt thereof in the manufacture of a medicament for use in the treatment of schizophrenia.

In yet another aspect described herein is a composition in a unit dosage form comprising (a) Meparfynol; (b) one or more additional agents selected from the group consisting of: (i) an NMDA/AMPA receptor modulator; (ii) a nootropic agent; (iii) an anti-depressive agent (eg SRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant; and (c) a buffer solution wherein Meparfynol and the agent are each present at an amount of from about 10 to 7000 mg, preferably 10 mg to about 1500 mg or more, more preferably from about 50 to 600 mg.

According to the disclosure, a method of treating schizophrenia in a subject in need of such treatment comprises administering to a subject in need thereof an effective amount of Meparfynol or a pharmaceutically acceptable salt thereof or a mixture thereof.

A method described herein of: (a) treating schizophrenia in an individual in need thereof; (b) slowing the progression of schizophrenia in an individual who has been diagnosed with schizophrenia; or (c) preventing or delaying development of schizophrenia in an individual who is at risk of developing schizophrenia, comprises administering to the individual an effective amount of Meparfynol or a pharmaceutically acceptable salt thereof.

A method of alleviating schizophrenia in a subject in need of such treatment comprises by administering to an individual an effective amount of Meparfynol or a pharmaceutically acceptable salt thereof. A method of alleviating schizophrenia in a subject in need of such treatment comprises by administering to an individual an effective amount of Meparfynol or a pharmaceutically acceptable salt thereof. A method of alleviating one or more symptoms associated with sensory processing impairment in a subject in need of such treatment comprises administering to an individual an effective amount of Meparfynol or a pharmaceutically acceptable salt thereof.

In another embodiment, described herein is a method of preventing and/or treating and/or delaying the onset and/or development of schizophrenia and/or decreasing the intensity or severity of the symptoms of schizophrenia in an individual and/or enhancing the quality of life of an individual (i) who is diagnosed with schizophrenia; (ii) who is considered at risk for developing schizophrenia (e.g., an individual whose one or more family members have had schizophrenia); (iii) who has been diagnosed as having a genetic mutation associated with schizophrenia or an individual who exhibits behaviour consistent with the onset of schizophrenia; (iv) who is genetically predisposed to developing schizophrenia; and/or (v) having a mutated or abnormal gene associated with schizophrenia (such as the NRG1 or DTNBP1 gene) but who has not been diagnosed with schizophrenia by administering to an individual an effective amount of Meparfynol or a pharmaceutically acceptable salt thereof.

Also described is a method of treating neuropsychiatric disorder in a patient comprising identifying a patient in need thereof and administering to said patient a therapeutically effective amount of a pharmaceutical composition comprising Meparfynol or a pharmaceutically acceptable salt thereof or a mixture thereof or mimetics or derivatives thereof, preferably Meparfynol, and a neuropsychiatric agent.

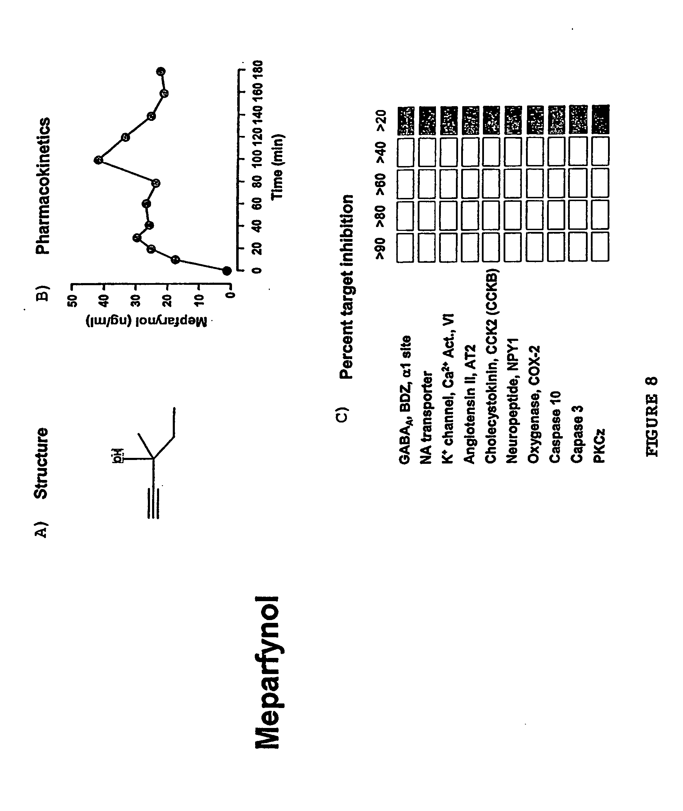

Thus, the invention provides the compound of the Formula II (3-methylpent-1-yn-3-ol) as depicted in Figure 8A or a pharmaceutically acceptable salt thereof for use in the treatment of schizophrenia in a subject in need of such treatment. The compound enhances a cognitive function and/or a sensory processing function.

Further provided is a pharmaceutical composition comprising a pharmaceutically acceptable carrier and, as an active ingredient, an effective amount of 3-methylpent-1-yn-3-ol for use in the treatment of schizophrenia.

Also described herein is a pharmaceutical composition comprising a pharmaceutically acceptable carrier and, as an active ingredient an effective amount of Meparfynol or a pharmaceutically acceptable salt thereof and one or more other agents selected from the group consisting of: (i) an NMDA/AMPA receptor modulator; (ii) a nootropic agent; (iii) an anti-depressive agent (eg an SRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant.

Also described herein is a pharmaceutical composition comprising a pharmaceutically acceptable carrier and, as an active ingredient an effective amount of Meparfynol or a pharmaceutically acceptable salt thereof and one or more other agents selected from the group consisting of: (i) an NMDA/AMPA receptor modulator; (ii) a nootropic agent; (iii) an anti-depressive agent (eg SRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant for use in the treatment of symptoms associated with psychosis, schizophrenia, a cognitive impairment disorder, a mood disorder, a nervous system disorder and/or a behavioural disorder in a subject in need of such treatment.

In yet another embodiment described herein is a pharmaceutical composition for the treatment of schizophrenia wherein the treatment comprises the simultaneous or sequential administration of Meparfynol or a pharmaceutically acceptable salt thereof and one and one or more additional agents selected from the group consisting of: (i) an NMDA/AMPA receptor modulator; (ii) a nootropic agent; (iii) an anti-depressive agent (eg SRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant.

The present disclosure also provides a process for making a pharmaceutical composition wherein the process comprises mixing Meparfynol or a pharmaceutically acceptable salt thereof and one or more additional agents selected from the group consisting of: (i) an NMDA/AMPA receptor modulator; (ii) a nootropic agent; (iii) an anti-depressive agent (eg an SRI); (iv) an Agonist/Antagonist/Partial Agonist/Partial Antagonist; (v) an anti-epileptic agent; (vi) (vi) a neuroleptic/neuropsychiatric agent; (vii) an atypical anti-psychotic agent; (viii) a cholinergic enhancer; and (ix) an attention stimulant/non-stimulant.

In an embodiment described herein, the agent is selected from the group of anti-psychotic agents consisting of chlorpromazine, thioridazine, mesoridazine, trifluoperazine, fluphenazine, perphenazine, loxapine, molindone, thiothixene, haloperidol, pimozide, clozapine, risperidone, olanzapine, quetiapine, sertindole, ziprasidone, donepezil (Aricept®)) and a selective metabotropic glutamate receptor agonist, such as a selective agonist for metabotropic gluatamate 2/3 (mGlu2/3) receptors (such as but not limited to those (Eli Lilly LY404039 and LY2140023) disclosed in Nature Reviews Drug Discovery (Dec 2007) 6: DOI10.1038/nrd2452).

The present disclosure provides a kit comprising: (a) Meparfynol or a pharmaceutically acceptable salt thereof or mixtures thereof; and (b) instructions for use in the treatment of schizophrenia.

The compound of the invention is 3-Methyl-1-pentyne-3-ol (MethylPentynol or Meparfynol).

3-Methyl-1-pentyne-3-ol is disclosed in US Patent No. 3966958 for use in low dose amounts in methods and compositions for increasing the feed intake of animals. This compound has been given the non-proprietary name, Meparfynol, which is used interchangeably with the names 3-Methyl-1-pentyne-3-ol and MethylPentynol throughout the specification. A process for preparing acetylenic alcohol compounds, such a Meparfynol, is disclosed in US Patent No. 2385547. Meparfynol can also be obtained commercially from Sigma-Aldrich Ltd, Airton Road, Tallaght, Dublin 24, Ireland and from FuRun Chemicals, No 88 Honghsi Road, Yubei District, Chongging 401147, China. Known properties in the published literature for Meparfynol includes its hynoptic and anticonvulsant effect (see, for example, Marley, E (Brit J Pharmacol (1959) 14: 284). By way of further example, Meparfynol is classified as a Sedative/Hypnotic in US Patent No. 7122198. There is no disclosure or suggestion in the any of the above references that the the compounds of U.S. Patent No. 3966958 may be used to treat symptoms associated with psychotic disorders, schizophrenic disorders and/or cognitive impairment disorders, a mood disorder, a nervious system disorder and/or a behavioural disorder.

The advantages of using Meparfynol, in the instant invention include the relatively nontoxic nature of the compound, the ease of preparation, the fact that the compound is well tolerated, and the ease of oral administration of the drug.

The present invention thus provides the compound Meparfynol or a pharmaceutically acceptable salts thereof for use in the treatment of subjects afflicted with schizophrenia. The compound Meparfynol or its salt may be prepared as a pharmaceutical composition particularly useful for the treatment of schizophrenia.

Thus, the subject invention also provides for a pharmaceutical composition comprising the compound Meparfynol or a pharmaceutically acceptable salt thereof and a pharmaceutically acceptable carrier for the treatment of schizophrenia.

Such compositions may comprise the compound Meparfynol or pharmaceutically acceptable salts thereof or mixtures thereof, together with pharmaceutically acceptable carriers and/or excipients. In the practice of this invention, pharmaceutically acceptable salts may be in either crystalline or amorphous form, and include solvates such as hydrates. Preferably the salt form is a Meparfynol carbamate salt form.

In one embodiment described herein, Meparfynol modulates a sensory processing function.

In another embodiment described herein, Meparfynol modulates a cognitive function.

In a further embodiment described herein, Meparfynol enhances a sensory processing function.

In yet another aspect described herein is a pharmaceutical composition comprising a pharmaceutically acceptable carrier and, as an active ingredient an effective amount of the compound Meparfynol or a pharmaceutically acceptable salt thereof and one or more additional agents selected from the group consisting of: (i) an NMDA/AMPA receptor modulator; (ii) a nootropic agent; (iii) an anti-depressive agent (eg an SRI); (iv) an Agonist/Antagonist/Partial Agonist/Partial Antagonist; (v) an anti-epileptic agent; (vi) an HDAC inhibitor; (vii) a neuroleptic/neuropsychiatric agent; (viii) an atypical anti-psychotic agent; (ix) a cholinergic enhancer; and (x) an attention stimulant/non-stimulant.

The present disclosure also provides a process for making a pharmaceutical composition wherein the process comprises mixing the compound Meparfynol or a pharmaceutically acceptable salt thereof or mixtures thereof and one or more additional agents selected from the group consisting of: (i) an NMDA/AMPA receptor modulator; (ii) a nootropic agent; (iii) an anti-depressive agent (eg an SRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant.

In an embodiment described herein, the anti-psychotic agent is selected from the group consisting of chlorpromazine, thioridazine, mesoridazine, trifluoperazine, fluphenazine, perphenazine, loxapine, molindone, thiothixene, haloperidol, pimozide, clozapine, risperidone, olanzapine, quetiapine, sertindole and ziprasidone, an acetylcholinesterase inhibitor, such as Donepezil or Aricept® which is disclosed and described in US Patent No. 4895841, WO2001/066114 and EP1311272B1, all of which are incorporated by reference in their entirety and a selective metabotropic glutamate receptor agonist, such as a selective agonist for metabotropic gluatamate 2/3 (mGlu2/3) receptors (such as but not limited to those (Eli Lilly LY404039 and LY2140023) disclosed in Nature Reviews Drug Discovery (Dec 2007) 6: DOI10.1038/nrd2452), which is also incorporated herein by reference.

In one embodiment described herein, the subject is a human being.

In another embodiment described herein the subject being treated is a mammal, including a human, in need of alleviation, or inhibition of schizophrenia.

In another embodiment described herein, the symptom of the disorder is associated with a sensory processing impairment.

In another embodiment described herein, the compound is administered orally, parenterally, rectally or transdermally.

In yet another embodiment described herein, the compound is administered in the range of from about 20 to 7000 mg, preferably from about 0.5mg to about 2000mg, even more preferably from about 0.5mg to about 1500 mg or more, yet more preferably from about 50 to 600 mg, even more preferably from about 20 and 200 mg, even more preferably from about 0.3 to about 100 mg/kg of body weight per, yet even more preferably from about 5-50mg/day.

In some embodiments described herein, suitable dosages of Meparfynol or a pharmaceutically acceptable salt thereof or mixtures thereof are typically about 0.05 to about 50 mg/day, for example from about 0.1 to about 40 mg/day or from about 0.2 to about 20 mg/day, preferably from about 4 to about 20 mg/day. Optionally, gradually increasing dosages can administered. That is, treatment can optionally start with relatively low doses which are incrementally increased until a maintenance dose is reached.

In another embodiment described herein, the pharmaceutical composition is in the form of a tablet, capsule, pill, powder or granule.

### DETAILED DESCRIPTION OF THE INVENTION

Various terms that will be used throughout the specification have meanings that will be well understood by the skilled addressee. However, for ease of reference, some of these terms will now be defined.

The present invention relates to a racemic compound of Meparfynol and to optically active isomers thereof. Meparfynol has an asymmetric carbon atom, and therefore, can exist either as racemic mixtures or as individual optical isomers (enantiomers). Meparfynol as described herein includes all possible stereoisomers of the compound, including compositions comprising the racemic mixture of the two enantiomers, as well as compositions comprising each enantiomer individually, substantially free of the other enantiomer.

It will be appreciated by those skilled in the art that Meparfynol may be derived from a prodrug. Examples of prodrugs include entities that have certain protected group(s) and which may not possess pharmacological activity as such, but may, in certain instances, be administered (such as orally or parenterally) and thereafter metabolised in the body to form the agent of the present invention which is pharmacologically active.

It will be further appreciated that certain moieties known as "pro-moieties", for example as described in "Design of Prodrugs" by H. Bundgaard, Elsevier, 1985 (the disclosure of which is hereby incorporated by reference), may be placed on appropriate functionalities of the compound of the present invention. Such prodrugs are also included within the scope of the present disclosure.

Typically Meparfynol, or pharmaceutically acceptable salts thereof will be prepared by chemical synthesis techniques in whole or in part.

Combinatorial libraries may be used to identify mimetics capable of acting in the same or a similar manner to Meparfynol, or pharmaceutically acceptable salts thereof.

As used herein, the term "mimetic" relates to any chemical which includes, but is not limited to, a peptide, polypeptide, antibody or other organic chemical which has the same qualitative activity or effect as Meparfynol, or pharmaceutically acceptable salts thereof. That is, a mimetic may be a functional equivalent of a known compound, such as Meparfynol, which is capable of inducing the improved pharmacological functional activity and/or behavioural response in a given subject.

The term "derivative" or "derivatised" as used herein includes chemical modification of Meparfynol, or pharmaceutically acceptable salts thereof. That is, a "derivative" may be a functional equivalent of Meparfynol, which is capable of inducing the improved pharmacological functional activity and/or behavioural response in a given subject. Illustrative of such chemical modifications would be replacement of hydrogen by a halo group, an alkyl group, an acyl group or an amino group.

The chemical modification of Meparfynol, or pharmaceutically acceptable salts thereof may either enhance or reduce hydrogen bonding interaction, charge interaction, hydrophobic interaction, Van Der Waals interaction or dipole interaction from the compound, such as Meparfynol, and its target.

In one embodiment described herein, Meparfynol, or pharmaceutically acceptable salts thereof may act as a model (for example, a template) for the development of other derivative compounds which are a functional equivalent of Meparfynol, which is capable of inducing the improved pharmacological functional activity and/or behavioural response in a given subject.

As used herein, the term "pharmaceutically acceptable salts" includes acid addition salts or addition salts of free bases. The term "pharmaceutically acceptable salts" of Meparfynol is also meant to include within its scope all the possible isomers and their mixtures, and any pharmaceutically acceptable metabolite, bioprecursor and/or pro-drug, such as, for example, a compound which has a structural formula different from Meparfynol, and yet is directly or indirectly converted *in vivo* into Meparfynol, upon administration to a subject, such as a mammal, particularly a human being.

The compound may be in the form of a pharmaceutically acceptable salt, such as an acid a ddition salt or a base salt, or a solvate thereof, including a hydrate thereof. For a review on suitable salts see Berge et al, J. Pharm. Sci., 1977, 66, 1-19. Suitable acid addition salts are formed from acids which form non-toxic salts and examples are the hydrochloride, hydrobromide, hydroiodide, sulphate, bisulphate, nitrate, phosphate, hydrogen phosphate, acetate, maleate, fumarate, lactate, tartrate, citrate, gluconate, succinate, saccharate, benzoate, methanesulphonate, ethanesulphonate, benzenesulphonate, p-toluenesulphonate and pamoate salts. Suitable base salts are formed from bases which form non-toxic salts and examples are the sodium, potassium, aluminium, calcium, magnesium, zinc and diethanolamine salts.

As used herein, the term "subject" refers to vertebrates, particularly members of the mammalian species.

As used herein, the term "psychosis" or "psychotic disorder" encompasses conditions which are listed in DSM-IV (Diagnostic and Statistical Manual of Mental Disorders - 4th Ed., Am. Psych. Association). Many psychotic disorders have similar or even the same symptoms of schizophrenia as described herein: psychotic disorder due to a general medical condition, delirium, or dementia; substance-induced psychotic disorder; substance-induced delirium; substance-induced persisting dementia; substance-related disorders; mood disorder with psychotic features; schizoaffective disorder; depressive disorder not otherwise specified; bipolar disorder not otherwise specified; mood disorder with catatonic features; schizophreniform disorder; brief psychotic disorder; delusional disorder; psychotic disorder not otherwise specified; pervasive developmental disorders (e.g., autistic disorder); childhood presentations combining disorganized speech (from a communication disorder) and disorganized behaviour (from attention-deficit/hyperactivity disorder); schizotypal disorder; schizoid personality disorder and paranoid personality disorder.

The term "cognitive function" refers to mental processes of an animal or human subject relating to information gathering and/or processing; the understanding, reasoning, and/or application of information and/or ideas; the abstraction or specification of ideas and/or information; acts of creativity, problem-solving, and possibly intuition; and mental processes such as learning, perception, and/or awareness of ideas and/or information. The mental processes are distinct from those of beliefs, desires, and the like. In some embodiments described herein, cognitive function may be assessed, and thus optionally defined, via one or more tests or assays for cognitive function. Non-limiting examples of a test or assay for cognitive function include CANTAB (see for example Fray et al. "CANTAB battery: proposed utility in neurotoxicology." Neurotoxicol Teratol. 1996; 18(4):499-504), Stroop Test, Trail Making, Wechsler Digit Span, or the CogState computerized cognitive test (see also Dehaene et al. "Reward-dependent learning in neuronal networks for planning and decision making." Prog Brain Res. 2000; 126:217-29; Iverson et al. "Interpreting change on the WAIS-III/WMS-III in clinical samples." Arch Clin Neuropsvchol. 2001; 16(2): 183-91; and Weaver et al. "Mild memory impairment in healthy older adults is distinct from normal aging." Brain Cogn. 2006;60(2): 146-55). A relatively new cognitive function endpoint test is the Neuropsychological test battery (NTB) test which may be used instead of the ADAS-cog "gold standard" test.

### Schizophrenia

According to the invention, the subject being treated is a mammal, including humans, in need of treatment, such as an alleviation, or inhibition of symptoms of a schizophrenic disorder.

By way of example, the subject may be a human: (i) who has been diagnosed with schizophrenia; (ii) who is suspected as having schizophrenia; (iii) who is considered at risk for developing schizophrenia (such as, for example, an individual whose one or more family members have had schizophrenia); (iv) who has been diagnosed as having a genetic mutation associated with schizophrenia; (v) who exhibits behaviour consistent with the onset of schizophrenia; (vi) who exhibits one or more symptoms associated with schizophrenia; (vii) who is genetically or otherwise predisposed to developing schizophrenia; (viii) who has a mutated or abnormal gene associated with schizophrenia (such as the NRG1 or DTNBP1 gene) but who has not been diagnosed with schizophrenia and/or who has one or more symptoms associated with schizophrenia.

Preferably the disorder to be treated is a disorder associated with schizophrenia.

As used herein, the term "schizophrenia" includes all forms and classifications of schizophrenia known in the art, including, but not limited to catatonic type, hebephrenic type, disorganized type, paranoid type, residual type or undifferentiated type schizophrenia and deficit syndrome and/or those described in American Psychiatric Association: Diagnostic and Statistical Manual of Mental Disorders Fourth Edition (DSM-IV), Washington D.C 2000 or in International Statistical Classification of Diseases and Related Health Problems, or otherwise known to those of skill in the art.

As used herein, the term "schizoaffective psychosis" means a disorder defined by DSM-IV characterized by the presence of symptoms of schizophrenia like delusions, hallucinations, disorganized speech, grossly disorganized or catatonic behaviour, negative symptoms together with mood symptoms like depression or mania.

As used herein, a subject "afflicted" with schizophrenia means the subject has been diagnosed with schizophrenia.

The clinical syndrome of schizophrenia comprises discrete clinical features. These clinical features comprise positive symptoms of schizophrenia which include delusions such as delusions of persecution, reference, thought withdrawal and thought insertion; hallucinations such as auditory, visual, olfactory, gustatory and tactile hallucinations; thought disorder; disorganized speech, and bizarre behaviour such as, for example, grossly disorganized or catatonic behaviour.

Negative, or deficit, symptoms of schizophrenia which include loss of motivation, restricted range of emotional experience and expression and reduced hedonic capacity, blunted affect, poverty of speech, anhedonia and asociality; and cognitive impairments with extensive variation from individuals. No single symptom is unique to schizophrenia and/or is present in every case.

Subjects are diagnosed as schizophrenic according to the DSM-IV criteria (APA,1994, Diagnostic and Statistical Manual of Mental Disorders (Fourth Edition), Washington,D.C.). An evaluation of memory enhancement in a subject having schizophrenic symptoms and having been treated using compositions and methods of the present embodiments can be assessed using the Scales for the Assessment of Negative Symptoms (SANS) or Positive and Negative Syndrome Scale (PANSS) (Andreasen, 1983, Scales for the Assessment of Negative Symptoms (SANS), Iowa City, Iowa; Kay et al, 1987, Schizophrenia Bulletin 13:261-276).

As used herein, "treatment" or "treating" is an approach for obtaining a beneficial or desired result, including clinical results. It is to be appreciated that all references herein to treatment includes curative, palliative, alleviative and/or prophylactic treatment. The term "treatment" also covers slowing the progression of and/or delaying the onset and/or development of a disease or a disordered state. Thus, the term "treatment" as used herein includes any care by procedures or applications to a subject, such as a mammal, and particularly a human, that are intended to: a) prevent the disease or disorder from occurring in a subject that may be predisposed to the disease/disorder, but has not yet been diagnosed with having it; b) inhibiting the disease/disorder, or condition, such as, for example, arresting its development; c) relieving the disease/disorder, or condition, such as, for example, causing regression of the disease/disorder, or condition; d) alleviating the symptoms associated with the disease/disorder and/or modulating the symptoms associated with the disease/disorder.

For purposes of this disclosure, beneficial or desired clinical results include an alleviation and/or diminishment and/or preventing a worsening of and/or a modulation of the symptoms associated with schizophrenia. Preferably, treatment with Meparfynol is accompanied by no or fewer side effects than those that are commonly associated with administration of anti-psychotic drugs, such as extrapyramidal side effects (such as rigidity, tremor), bradykinesia (slow movement), and bradyphrenia (slow thought), acute dystonia (such as involuntary muscle contraction), acute dyskinesia, and tardive dyskinesia (such as involuntary movements).

As used herein, the term "modulate" or "modulation" refers to any change in, for example, a pharmacological functional activity and/or a behavioural response relative to a normal or reference level of a pharmacological functional activity and/or a behavioural response under the same conditions. For example, the pharmacological functional activity and/or a behavioural response can be greater or lesser than that of a reference entity.

As used herein, the various forms of the term "modulate" include potentiating (such as, for example, increasing or upregulating or improving or enhancing a particular behavioural response and/or pharmacological functional activity) and inhibiting (such as, for example, decreasing or downregulating a particular behavioural response and/or pharmacological function activity). The potentiating and/or inhibiting can take place directly and/or indirectly and/or competitively and/or non-competitively (such as, for example, working at a site which is distal to receptor rather than at a receptor site itself). By way of example, the behavioural response and/or pharmacological function activity of an entity may be modulated indirectly, such as, for example, by modulating the activity of a molecule, such as an effector molecule, that is upstream or downstream of the entity in a signal transduction pathway involving that entity. By way of example, modulating a cognitive function by improving or enhancing a cognitive function includes "promoting" a cognitive function (affecting impaired cognitive function in the subject so that it more closely resembles the function of an aged-matched normal, unimpaired subject, including affecting states in which cognitive function is reduced compared to a normal subject) and "preserving" cognitive function (affecting normal or impaired cognitive function such that it does not decline or does not fall below that observed in the subject upon first presentation or diagnosis, such as, for example, to the extent of expected decline in the absence of treatment).

As used herein, the term "enhanced cognition" means an improvement in a mental function, such as learning or memory.

As used herein, the term "enhanced sensory processing function" includes an improvement in the organization of sensory input for use. The "use" may be a perception of the body or the world, or an adaptive response, or a learning process, or the development of some neural function. Through a sensory processing function, the many parts of the nervous system work together so that a person can interact with the environment effectively and experience appropriate satisfaction. Essentially, it is a form of working memory. A series of tests may be used to measure the status of a sensory processing function in a subject.

As used herein, the term "sensory processing impairment" refers to an irregularity or disorder or upset in brain function that makes it difficult to integrate sensory input effectively. In this regard, sensory impairment may be present in motor, learning, social/emotional, speech/language or attention disorders.

### Combination therapy

The compound of the present invention is also useful in association with or in combination with other agents and/or treatments that are useful in, for example, improving cognitive function in pathological conditions symptoms associated with schizophrenia. Combination therapy can also involve, for example, separate, simultaneous or sequential delivery of the two active agents.

The combination therapy may be of one of the above with Meparfynol or a pharmaceutically acceptable salt thereof as described herein to improve the condition of the subject or patient. Non-limiting examples of combination therapy include the use of lower dosages of the above additional agents, or combinations thereof, which reduce side effects of the agent or combination when used alone. For example, an anti-depressant agent like fluoxetine or paroxetine or sertraline may be administered at a reduced or limited dose, optionally also reduced in frequency of administration, in combination with a nootropic agent, which are used in combination with Meparfynol or a pharmaceutically acceptable salt thereof or mixtures thereof.

In light of the positive recitation (above and below) of combinations with alternative agents to treat conditions disclosed herein, the disclosure includes embodiments with the explicit exclusion of one or more of the alternative agents. As would be recognized by the skilled person, a description of the whole of a plurality of alternative agents necessarily includes and describes subsets of the possible alternatives, or the part remaining with the exclusion of one or more of the alternatives.

The present disclosure contemplates the use of Meparfynol or a pharmaceutically acceptable salt thereof in combination(s) with other agents to treat patients suffering from schizophrenia. By way of example, other therapeutics that may be administered with Meparfynol or a pharmaceutically acceptable salt thereof or mixtures include: (i) an NMDA/AMPA receptor modulator; (ii) a nootropic agent; (iii) an anti-depressive agent (eg an SRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent and/or (viii) a cholinergic enhancer and/or (ix) an attention stimulant/non-stimulant. Meparfynol or a pharmaceutically acceptable salt thereof also may be used in combination with behavioural therapy.

Examples of NMDA receptor modulators that are contemplated by the present disclosure include memantine and amantadine.

Examples of nootropic agents that are contemplated by the present disclosure include piracetam (nootropil).

Examples of serotonin re-uptake inhibitors (SRIs) that are contemplated by the present disclosure include clomipramine, fluoxetine, fluvoxamine, sertraline, paroxetine, citalopram, escitalopram, venlafaxine, mirtazepine, duloxetine and mixtures thereof.

Examples of Agonist/Antagonist/Partial Agonist/Partial Antagonist that are contemplated by the present disclosure include 5HT2/3/4/5/6 receptor agonists, antagonistis, partial agonists and antagonistis. One particular preferred 5HT6 antagonists is SB-271046, currently being developed by GSK.

Examples of anti-epileptic agents that are contemplated by the present disclosure include valproate, divalproex, gabapentin, topiramate, leviracetam, lamotrigine, carbamazapine, oxcarbamazepine, tiagabine, zonisamide, clonzaepam, pregabalin, zarontin and mixtures thereof.

Examples of attention stimulants/non-stimulants that are contemplated by the present disclosure include dextroamphetamine, methylphenidate, adderall, adderall XR, concerta, focalin, and strattera.

Examples of atypical antipsychotics that are contemplated by the present disclosure include risperidone, olanzepine, quetiapine, ziprasidone, and aripiprazole.

Examples of cholinergic enhancers that are contemplated by the present disclosure include aricept (donepezil), excelon, reminyl (galantamine), and mestinon.

### NMDA Receptor Modulators and AMPA Receptor Modulators

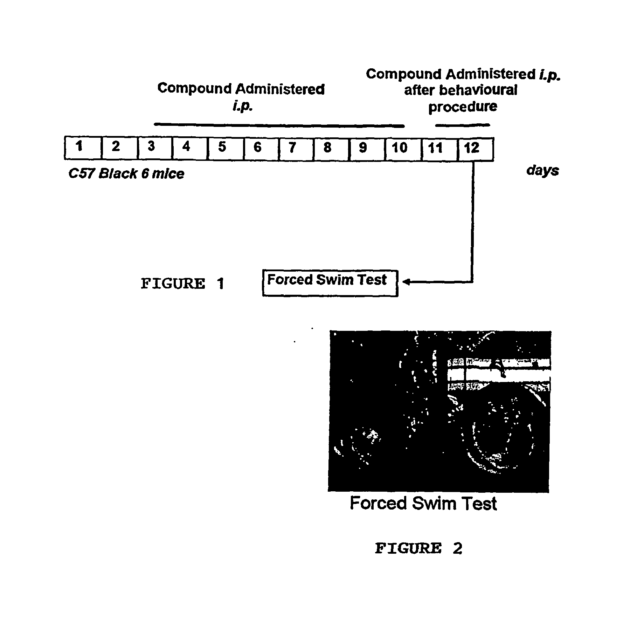

Information storage in the brain underlies learning and memory. One of the cellular mechanisms for information storage is the phenomenon of long-term potentiation (LTP) of synaptic transmission at glutamatergic synapses. The mechanisms underlying LTP formation involve the main types of ionotropic glutamatergic receptors, the AMPA and the NMDA receptors (FIG. 1 from Lynch, G., 2002, Nature Neurosci. 5:1035-1038). LTP is elicited by delivering brief pulses of high frequency stimulation to monosynaptic connections in several defined anatomical pathways in the hippocampus and other brain structures. High frequency stimulation activates AMPA receptors and produces sufficient postsynaptic depolarization to release the NMDA receptor channel from its voltage-dependent magnesium blockade resulting in an influx of calcium and a modification of the AMPA receptors. Increased intracellular calcium is depicted as triggering signaling pathways that modify the properties of cell adhesion molecules, thereby providing for structural modifications of synaptic contacts. Signaling pathways are depicted as modifying the activity of transcription factors such as the calcium/calmodulin response element binding (CREB) protein, resulting in transcriptional responses leading to long-term modifications of cell function. Activation of both AMPA and NMDA receptors is therefore key in LTP formation and, thereby, in memory formation.

The N-methyl-D-aspartate (NMDA) receptor is a postsynaptic, ionotropic receptor which is responsive to the amino acids glutamate and glycine, and the synthetic compound NMDA. The NMDA receptor controls the flow of both divalent (Ca ++) and monovalent (Na+, K+) ions into the postsynaptic neural cell through a receptor associated channel (Foster et al., Nature 1987; 329: 395-396; Mayer et al., Trends in Pharmacol. Sci. 1990; 11: 254-260). There is also a strychnine insensitive glycine binding site proximate to the NMDA/glutamate/aspartate binding site. It has been shown that glycine site agonists are necessary for channel function and that low intrinsic activity partial agonists, such as HA-966 (3-amino-1-hydroxypyrrolid-2-one; Merck) behave as functional NMDA antagonists in the presence of sufficient agonist.

U.S. Patent No. 5,086, 072, issued to Trullas et al., described the use of 1-aminocyclopropanecarboxylic acid (ACPC), which was thought to be a functional antagonist of the NMDA site *via* its activity as a partial agonist of the strychnine-insensitive glycine binding site, to treat mood disorders including major depression, bipolar disorder, dysthymia and seasonal affective disorder. It was also therein described that intraperitoneally-administered ACPC, *via* this functional antagonism, mimicked the actions of clinically effective antidepressants in animal models. However, it was subsequently demonstrated that sustained exposure to ACPC in fact attenuated its protective effects and increased the receptors'sensitivity to glutamate (Fossom et al., Mol. Pharmacol. 1995; 48: 981-87), and that ACPC is actually a full glycine agonist (Nathum-Levy et al., Mol. Pharmacol. 1999; 56: 1207-18).

International (PCT) Application WO00/02551, to Mueller et al., described novel compounds active at both the serotonin reuptake site and the NMDA receptor (i.e., inhibition of both sites) that can be used to treat different types of disorders such as depression, obsessive-compulsive disorders (OCD), sleep disorders, sexual dysfunction, and eating disorders. According to this PCT application (WO 00/02551), potent activity at the serotonin reuptake site was favored, while an intermediate activity at the NMDA receptor was also favoured. Too potent an activity at the NMDA receptor is less preferred because of possible PCP-like side effects.

A number of preclinical experiments have been reported as evidence that glutamate and the NMDA receptor may be involved in the etiology of depressive disorders (Skolnick, Eur J Pharmacol. 1999; 375: 31-40; and Skolnick et al., Pharmacopsychiatry. 1996; 29: 1,23-6). NMDA receptor antagonists have been shown to exhibit antidepressant like activity in animal models of depression (Rogoz et al., Neuropharmacology. 2002; 42 (8): 1024-30). Memantine, a moderate affinity, uncompetitive N-methyl-D-aspartate (NMDA) receptor antagonist, reduces glutamatergic output *via* open-channel block of the NMDA receptor-associated ion channel thereby reducing or preventing neuronal damage from excitotoxicity. See, e. g., U.S. Patents 6, 071,966; 6,034, 134; and 5,061, 703, all which is incorporated herein by reference.

The NMDA receptors exhibit a variety of modulatory sites and, in particular, exhibit a binding site for the amino acid glycine. Several compounds acting at the glycine site of the NMDA receptor have also been shown to facilitate LTP formation and have been proposed as cognitive enhancers such as D-serine and D-cycloserine, for example. Further, inhibitors of glycine uptake exert similar effects as glycine, facilitate LTP formation, and are proposed as cognitive enhancers. Drugs acting as positive modulators of NMDA receptors are termed "Nemdakines."

A category of drugs, termed "Ampakines" acts as positive modulators of AMPA receptors (PARMs). Ampakines have been proposed as cognitive enhancers due to their ability to facilitate LTP induction and to facilitate learning in a variety of tasks in mammals and humans. Ampakines are in clinical trials to treat various indications including mild cognitive impairment (MCI) associated with aging.

A variety of accepted tests are used to determine whether a given agent is a positive modulator of an AMPA or an NMDA receptor. The primary *in vitro* assay is measurement of the enlargement of the excitatory postsynaptic potential *(EPSP) in vitro* brain slices, such as rat hippocampus brain slices. Modulators useful in the present embodiments are agents that cause an increased ion flux through the AMPA or NMDA receptor complex channels. Increased ion flux is typically measured as at least a 10% increase in decay time, amplitude of the waveform and/or the area under the curve of the waveform and/or a decrease of at least 10% in rise time of the waveform, for example.

Thus, compositions comprising Meparfynol and/or salts thereof in combination with ampakines and/or nemdakines may improve the condition of patients with cognitive impairment by improving long-term potentiation of synaptic transmission.

Examples of an ampakine include but are not limited to a azepine, a benzamide, benzoylpiperidine, benzoylpyrrolidine, benzoxazine, benzothiadiazide, benzothiadiazine, biarylpropylsulfonamide, pyrrolidinone, pyrroline, tetrahydropyridine, phenoxyacetamide, sulfur-containing organic nitrate ester, or a lectin. In one embodiment described herein, the ampakine functional moiety is derived from the benzoylpiperidine, CX546, and in a further embodiment described herein of a fusion molecule, the ampakine functional moiety is derived from the biarylpropylsulfonamide derivative, LY404187-NH₂. The nemdakine functional moiety of a fusion molecule is derived from a nemdakine such as L-alanine, D-alanine, D-cycloserine, N-methylglycine, L-serine, D-serine, N,N,N- trimethylglycine, 3-amino-1-hydroxypyrrolid-2-one(HA966),(R)-(N-[3-(4'-fluorophenyl)-3-(4'phenylphenoxy) propyl]) sarcosine (ALX5407), N-methyl-N-[3-[(4- trifluoromethyl)phenoxy]-3-phenyl-propyl] glycine (ORG 24598), a polyamine, or a neurosteroid. In one embodiment of a fusion molecule, the nemdakine functional moiety is derived from D-serine or L-serine. Examples of fusion molecules provided herein include: LB-217-lc having an IUPAC name of (R)-2-amino-3-[1-(2,3-dihydro- benzo[1,4]dioxine-6-carbonyl)-piperidin-4-yl]-2-hydroxymethyl-propionic acid, LB-253-4c having an IUPAC name of 2-ammo-2-hydroxymethyl-6-{4'-[1-methyl-2-(propane-2-sulfonylamino)-ethyl]-biphenyl-4-ylamino}-hexanoic acid, and LB-302 having an IUPAC name of 2-amino-3-(1-benzyl-1,2,3,6-tetrahydro-pyridin-4- yl)-2-hydroxymethyl-propionic acid.

Amantadine was shown to have NMDA non-competitive inhibitor activity at doses routinely used for Alzheimer's and Parkinson's disease (Kornhuber et al, J Neural Transm Suppl., 43: 91-104 (1994)), without having any psychomimetic side effects. On the basis of that, a double blind placebo controlled trial of Amantadine was carried out in autistic children. While it was tolerated, it was noted to have a modest, at best, effect on irritability and hyperactivity. This effect may be due to amantadine's low affinity for the NMDA receptor.

### Memantine

Examples of a nemdakine include but are not limited to Memantine. Memantine (Namenda™) (1-amino-3,5-dimethyl adamantane), which is disclosed, e.g., in U.S. Patent Nos. 4,122,193; 4,273,774; and 5,061,703, is a systemically-active uncompetitive NMDA receptor antagonist having low to moderate affinity for the receptor and strong voltage dependency and rapid blocking/unblocking kinetics. Memantine hydrochloride is currently available in the U.S. and in over 42 countries worldwide. It is approved for the treatment of moderate to severe Alzheimer's disease (AD) in the United States at a dose of up to 20 mg/day (5-10 mg BID). It has been hypothesized that Memantine may not only be effective for the treatment of Alzheimer's disease (as well as Parkinson's and other neurological diseases), but may also be effective for the treatment of autism, Attention-Deficit/Hyperactivity Disorder (ADHD) and other autistic spectrum disorders. In the United States, the trade name for memantine is Namendag, in Germany as Akatinol and Auxura, and Ebixa in the European Union. Memantine is also widely used for the treatment of Parkinson's disease, dementia, and spasticity in Germany, and has been approved for the treatment of moderately severe to severe Alzheimer's disease in the European Union and in moderate to severe Alzheimer's disease the United States. It is also currently being evaluated in the United States in clinical studies of patients with painful diabetic neuropathy.

The approval by the FDA of Memantine for the treatment of Alzheimer's disease was based on three randomized placebo-controlled trials that showed significant improvements in cognitive, functional and global endpoints in this population (Tariot et al., JAMA. 2004;291:317-24, Reisberg et al, N Engl J Med., Apr 3; 348(14): 1333-41 (2003), Winblad et al., Int J Geriatr Psychiatry, 14(2): 135-46 (1999)). Similar results were seen in two trials in vascular dementia (Wilcock et al., Int Clin Psychopharmacol., 17(6): 297-305(2002), Orgogozo et al., Stroke,33: 1834-9 (2002)). Memantine has been used in Germany for a variety of neurological syndromes and cognitive deficits since 1982 with good tolerability. In animal models, memantine has been shown to prolong the duration of long term potentiation *in vivo* and to improve learning and memory. (Zajaczkowski et al., Eur J Pharmacol., 296(3): 239-46 (1996)). Neuroprotection has been demonstrated in animals (Danysz et al., Amino Acids., 19(1): 167-72 (2000)).

Memantine has moderate affinity for the NMDA receptor, with strong voltage dependency and rapid blocking and unblocking properties (Mobius, Int J Geriatr Psychiatry 18(Suppl 1): S47-54 (2003)). It seems to block the sustained activation at micro-molar concentrations of glutamate under pathological conditions but to rapidly leave the NMDA channel upon transient physiological activation at milli-molar concentrations of synaptic glutamate (Parsons et al., Amino Acids. 19(1): 157-66 (1993)). It occupies approximately five out of the six ion channels in the receptor, allowing the sixth one to remain free under resting conditions and available for physiological transmission. (Blanpied et al., J Neurophysiol 77(1): 309-23 (1997)). These features are thought to form the basis for the lack of psychomimetic side effects seen with PCP and PCP-like substances. Memantine undergoes little metabolism, with the majority (57-82%) of an administered dose excreted unchanged in urine. The remainder is converted primarily into, three metabolites: the N-gludantan conjugate, 6-hydroxy memantine, and 1-nitroso-deaminated memantine. These metabolites have minimal NMDA receptor antagonist activity.

### Nootropic Agents

Nootropic agents refer to drugs that are thought to enhance cognitive function and/or mental activity. One exemplary nootropic agent is Piracetam™ (see U.S. Patent 4,620,973).

In some cases, Meparfynol or a pharmaceutically acceptable salt thereof, optionally in combination with one or more other neurogenic agents, results in improved efficacy, fewer side effects, lower effective dosages, less frequent dosing, and/or other desirable effects relative to use of the neurogenesis modulating agents individually (such as at higher doses), due, for example, to synergistic activities and/or the targeting of molecules and/or activities that are differentially expressed in particular tissues and/or cell-types.

A nootropic agent for use in embodiments of the disclosure may be an agent suitable for *in vivo* or *in vitro* use as described herein. Alternatively, a nootropic agent may be unsuitable for *in vivo* application but suitable for *in vitro* use, such as the treatment of cells outside the subject from which they were obtained or the treatment of cells of a cell line. The treatment of cells *in vitro* may of course be part of *an ex vivo* procedure wherein the cells are returned to the subject (from which they were obtained or to a subject of the same species) after the treatment.

Preferably the nootropic agent of the disclosure is a racetam, a class of nootropic drugs where each class member contains a pyrrolidine nucleus as a common feature. Non-limiting examples of racetams include water-soluble racetams, such as Piracetam or Oxiracetam, and fat-soluble racetams, such as Aniracetam or Pramiracetam. Other non-limiting examples of racetams include Etiracetam (CAS RN 33996-58-6), Levetiracetam, Nefiracetam, Rolziracetam (CAS RN 18356-28-0), Nebracetam (CAS RN 97205-34-0 or 116041-13-5), Fasoracetam (CAS RN 110958-19-5), Brivaracetam (CAS RN 357336-20-0), and Seletracetam (CAS RN 357336-74-4).

A nootropic agent for use in embodiments of the disclosure include Piracetam (Nootropil), or 2-oxo-1-pyrrolidineacetamide, which is referenced by Chemical Abstracts Service Registry Number (CAS RN) 7491-74-9; Aniracetam, or 1-(4-methoxybenzoyl)-2-pyrrolidinone, (CAS RN 72432-10-1); 3-hydroxy aniracetam or (R)-3 -Hydroxy-1-(4-methoxybenzoyl)-2-pyrrolidinone (CAS RN 78340-51-9); Oxiracetam, or 4-hydroxy-2-oxo-1-pyrrolidineacetamide, (CAS RN 62613-82-5); (+-)-oxiracetam or (+-)-4-hydroxy-2-oxo-1-pyrrolidineacetamide (CAS RN 68567-97-5); Pramiracetam, or N-(2-(bis(1-methylethyl)amino)ethyl)-2-oxo-1 -pyrrolidineacetamide, (CAS RN 68497-62-1); pramiracetam sulfate (CAS RN 72869-16-0); pramiracetam hydrochloride (CAS RN 75733- 50-5); Pyritinol (Enerbol), or 3,3'-(dithiodimethylene)bis(5-hydroxy-6-methyl-4-pyridinemethanol), (CAS RN 1098-97-1); pyritinol dihydrochloride (CAS RN 10049-83-9); Ergoloid mesylates (Hydergine), or an equi-proportional mixture of dihydroergocornine mesylate, dihydroergocristine mesylate, and ratio of dihydro-alpha-ergocryptine mesylate to dihydro-beta-ergocryptine mesylate is 1.5-2.5:1, (CAS RN 8067-24-1); Galantamine, or (4aS,6R,8aS)-4a,5,9,10,11,12-Hexahydro-3-methoxy-11-methyl-6H-benzofuro[3a,3,2-efJ[2]benzazepin-6-ol, (CAS RN 357-70-0, see also Czollner, et al. ARKTVOC 191-200 (2001)); galantamine hydrobromide (CAS RN 69353-21-5); Selegiline, or N-methyl-N-(1-methyl-2-phenyl-ethyl)-prop-2-yn-1 -amine, (CAS RN 14611-51-9, see also Torok et al., Acta Pharm Hung. (1992) 62(5):201-11); selegiline hydrochloride (CAS RN 14611-52-0); Centrophenoxine (Lucidril), also known as meclofenoxate hydrochloride or (p-Chlorophenoxy)acetic acid 2-(dimethylamino)ethyl ester hydrochloride, (CAS RN 3685-84- 5); Desmopressin (DDAVP), or 1-[[13-benzyl-10-(2-carbamoylethyl)-7-(carbamoylmethyl)- 16-[(4-hydroxyphenyl)methyl]-6,9, 12, 15,18-pentaoxo- 1,2-dithia-5,8, 11 , 14, 17- pentazacycloicos-4-yl]carbonyl]-N-[1-(carbamoylmethylcarbamoyl)-4-guanidino-butyl]- pyrrolidine-2-carboxamide, (CAS RN 16679-58-6); desmopressin acetate (CAS RN 62288- 83-9 or 62357-86-2); Nicergoline, or (+)-10-Methoxy-1,6-dimethylergoline-8-beta-methanol 5-bromonicotinate, (CAS RN 27848-84-6); nicergoline tartrate (CAS RN 32222-75-6); Vinpocetine, also known as 3-alpha,16-alpha-apovincaminic acid ethyl ester or (3alpha,16alpha)-eburnamenine-14-carboxylic acid, ethyl ester, (CAS RN 42971-09-5); Picamilon, or monosodium 4-((3-pyridinylcarbonyl)amino)butanoate, (CAS RN 62936-56-5); Vasopressin, a nine amino acid peptide secreted from the posterior pituitary, (CAS RN 11000-17-2); Milacemide, or 2-(pentylamino)acetamide, (CAS RN 76990-56-2); milacemide hydrochloride (CAS RN 76990-85-7); FK-960, represented by the following structure: FK-962, or N-(1-acetylpiperidin-4-yl)-4-fluorobenzamide), is a derivative of FK-960 (see Tokita et al. "FK962, a novel enhancer of somatostatin release, exerts cognitive- enhancing actions in rats." Eur J Pharmacol. (2005) 527(1 -3): 111-20); SGS-111, represented by the following structure: M6, or cyclo-(Pro-Gly), a metabolite of SGS-111; Levetiracetam, or (S)- alpha-ethyl-2-oxo-1-pyrrolidineacetamide, (CAS RN 102767-28-2); Nefiracetam, or N-(2,6-dimethylphenyl)-2-oxo- 1 -pyrrolidineacetamide, (CAS RN 77191 -36-7); Hyperzine A (CAS RN 120786-18-7); or an ergot alkaloid (CAS RN 12126-57-7).

In some embodiments of the disclosure, however, the use of a nootropic agent, nootropic ligand, or racetam as described herein excludes the compound known as coluracetam, or N-(2,3-dimemyl-5,6,7,8-tetrahydrofuro(2,3-b)quinolin-4-yl)-2-(2-oxopyrrolidin-1-yl) acetamide, (CAS RN 135463-81-9). Without being bound by theory, and offered to improve the understanding of the invention, it has been reported that the racetams function *via* activation of glutamate receptors that are colocalized with cholinergic receptors, which increases activity of of the latter.

Also without being bound by theory, and offered to improve the understanding of the invention, it is thought that the neurogenic action of nootropic agents may be through AMPA (alpha-amino-3-hydroxy-5-methyl-4-isoxazolepropionic acid) receptor potentiation or sensitization. Thus in an additional aspect, the disclosure provides for the use of Meparfynol or a pharmaceutically acceptable salt thereof in combination with a nootropic agent in combination with AMPA, or other AMPA receptor agonist. The nootropic agent may act as a potentiator of the action, or activity, of AMPA or another ligand. In some embodiments described herein, the nootropic agent may be similar to the known AMPA potentiator, 4-[2-(phenylsulfonylamino)ethylthio]-2,6-difluoro-phenoxyacetamide (PEPA).

A nootropic agent as described herein includes pharmaceutically acceptable salts, derivatives, prodrugs, and metabolites of the agent. Methods for preparing and administering salts, derivatives, prodrugs, and metabolites of various agents are well known in the art.

As described herein, Meparfynol or a pharmaceutically acceptable salt thereof or mixtures thereof in combination with a nootropic agent, optionally in combination with one or more other neurogenic agents, is administered to an animal or human subject. A combination may thus be used to treat schizophrenia in a subject in need of such treatment.

### Anti-depressant agents (eg SSRI (selective serotonin reuptake inhibitors)

The disclosure includes methods for treating depression linked with schizophrenia in a subject in need of such treatment with Meparfynol or a pharmaceutically acceptable salt thereof. In some embodiments described herein, a method may comprise the use of a combination of Meparfynol or a pharmaceutically acceptable salt thereof and one or more agents reported as anti-depressant agents as known to the skilled person.

Examples of such agents include an SSRI (selective serotonine reuptake inhibitor), such as fluoxetine (Prozac®; described, e.g., in U.S. Pat. 4,314,081 and 4,194,009), citalopram (Celexa; described, e.g., in U.S. Pat. 4,136,193), escitalopram (Lexapro; described, e.g., in U.S. Pat. 4,136,193), fluvoxamine (described, e.g., in U.S. Pat. 4,085,225) or fluvoxamine maleate (CAS RN: 61718-82-9) and Luvox®, paroxetine (Paxil®; described, e.g., in U.S. Pat. 3,912,743 and 4,007,196), or sertraline (Zoloft®; described, e.g., in U.S. Pat. 4,536,518), or alaproclate; the compound nefazodone (Serozone®; described, e.g., in U.S. Pat. 4,338,317); a selective norepinephrine reuptake inhibitor (SNRI) such as reboxetine (Edronax®), atomoxetine (Strattera®), milnacipran (described, e.g., in U.S. Pat. 4,478,836), sibutramine or its primary amine metabolite (BTS 54 505), amoxapine, or maprotiline; a selective serotonin & norepinephrine reuptake inhibitor (SSNRI) such as venlafaxine (Effexor; described, e.g., in U.S. Pat. 4,761,501), and its reported metabolite desvenlafaxine, or duloxetine (Cymbalta; described, e.g., in U.S. Pat. 4,956,388); a serotonin, noradrenaline, and dopamine "triple uptake inhibitor", such as DOV 102,677 (see Popik et al. "Pharmacological Profile of the "Triple"Monoamine Neurotransmitter Uptake Inhibitor, DOV 102,677." Cell MoI Neurobiol. 2006 Apr 25; Epub ahead of print), DOV 216,303 (see Beer et al. "DOV 216,303, a "triple" reuptake inhibitor: safety, tolerability, and pharmacokinetic profile." J Clin Pharmacol. 2004 44(12): 1360-7), DOV 21,947 ((+)-1-(3,4-dichlorophenyl)-3-azabicyclo-(3.1.0)hexane hydrochloride), see Skolnick et al. "Antidepressant-like actions of DOV 21,947: a "triple" reuptake inhibitor." Eur J Pharmacol. 2003 461(2-3):99-104),NS-2330 or tesofensine (CAS RN 402856-42-2), or NS 2359 (CAS RN 843660-54-8); and agents like dehydroepiandrosterone (DHEA), and DHEA sulfate (DHEAS), CP-122,721 (CAS RN 145742-28-5).

Additional examples of such agents include a tricyclic compound such as clomipramine, dosulepin or dothiepin, lofepramine (described, e.g., in 4,172,074), trimipramine, protriptyline, amitriptyline, desipramine(described, e.g., in U.S. Pat. 3,454,554), doxepin, imipramine, or nortriptyline; a psychostimulant such as dextroamphetamine and methylphenidate; an MAO inhibitor such as selegiline (Emsam®); an ampakine such as CX516 (or Ampalex, CAS RN: 154235-83-3), CX546 (or 1-(1,4- benzodioxan-6-ylcarbonyl)piperidine), and CX614 (CAS RN 191744-13-5) from Cortex Pharmaceuticals; a VIb antagonist such as SSR149415 ((2S,4R)-1-[5-Chloro-1-[(2,4-dimethoxyphenyl)sulfonyl]-3 -(2-methoxy-phenyl)-2-oxo-2,3 -dihydro- 1 H-indol-3 -yl] - 4- hydroxy-N,N-dimethyl-2 -pyrrolidine carboxamide),[1 -(beta-mercapto-beta,beta-cyclopentamethylenepropionic acid), 2-0- ethyltyrosine, 4-valine] arginine vasopressin (d(CH2)5[Tyr(Et2)]VAVP (WK 1-1), 9-desglycine [1-(beta-mercapto-beta,beta-cyclopentamethylenepropionic acid), 2-0-ethyltyrosine, 4-valine] arginine vasopressin desGly9d(CH2)5 [Tyr(Et2)]-VAVP (WK 3-6), or 9-desglycine [1-(beta-mercapto-beta,beta- cyclopentamethylenepropionic acid),2-D-(O-ethyl)tyrosine, 4-valine] arginine vasopressin des Gly9d(CH2)5[D- Tyr(Et2)] VAVP (AO 3-21); a corticotropin-releasing factor (CRP) R antagonist such as CP- 154,526 (structure disclosed in Schulz et al. "CP-154,526: a potent and selective nonpeptide antagonist of corticotropin releasing factor receptors." Proc Natl Acad Sci USA. 1996 93(19): 10477-82), NBI 30775 (also known as R121919 or 2,5-dimethyl-3-(6-dimethyl-4- methylpyridin-3-yl)-7-dipropylaminopyrazolo[1,5-a]pyrimidine), astressin (CAS RN 170809- 51 -5), or a photoactivatable analog thereof as described in Bonk et al. "Novel high-affinity photoactivatable antagonists of corticotropin-releasing factor (CRF)" Eur. J. Biochem. 267:3017-3024 (2000), or AAG561 (from Novartis); a melanin concentrating hormone (MCH) antagonist such as 3,5-dimethoxy-N-(1-(naphthalen-2-ylmethyl)piperidin-4-yl)benzamide or (R)-3,5-dimethoxy-N-(1 -(naphthalen-2-ylmethyl)-pyrrolidin-3-yl)benzamide (see Kim et al. "Identification of substituted 4-aminopiperidines and 3-aminopyrrolidines as potent MCH-R1 antagonists for the treatment of obesity." Bioorg Med Chem Lett. 2006 JuI 29; [Epub ahead of print] for both), or any MCH antagonist disclosed in U.S. Patent 7,045,636 or published U.S. Patent Application US2005/0171098.

Further examples of such agents include a tetracyclic compound such as mirtazapine (described, e.g., in U.S. Pat. 4,062,848; see CAS RN 61337-67-5; also known as Remeron, or CAS RN 85650-52-8), mianserin (described, e.g., in U.S. Pat. 3,534,041), or setiptiline.

Further examples of such agents include agomelatine (CAS RN 138112-76-2), pindolol (CAS RN 13523-86-9), antalarmin (CAS RN 157284-96-3), mifepristone (CAS RN 84371-65-3), nemifitide (CAS RN 173240-15-8) or nemifitide ditriflutate (CAS RN 204992-09-6), YKP-IOA or R228060 (CAS RN 561069-23-6), trazodone (CAS RN 19794-93-5), bupropion (CAS RN 34841-39-9 or 34911-55-2) or bupropion hydrochloride (or Wellbutrin, CAS RN 31677-93-7) and its reported metabolite radafaxine (CAS RN 192374-14-4), NS2359 (CAS RN 843660-54-8), Org 34517 (CAS RN 189035-07-2), Org 34850 (CAS RN 162607-84-3), vilazodone (CAS RN 163521-12-8), CP- 122,721 (CAS RN 145742-28-5), gepirone (CAS RN 83928-76-1), SR58611 (see Mizuno et al. "The stimulation of beta(3)-adrenoceptor causes phosphorylation of extracellular signal-regulated kinases 1 and 2 through a G(s)- but not G(i)-dependent pathway in 3T3-L1 adipocytes." Eur J Pharmacol. 2000 404(1 -2):63-8), saredutant or SR 48968 (CAS RN 142001-63-6),PRX-00023(N-{3-[4-(4cyclohexylmethanesulfonylaminobutyl) piperazin-1-yl]phenyl}acetamide, see Becker et al. "An integrated in silico 3D model-driven discovery of a novel, potent, and selective amidosulfonamide 5-HT1 A agonist (PRX-00023) for the treatment of anxiety and depression." J Med Chem. 2006 49(11):3116-35), Vestipitant (or GW597599, CAS RN 334476-46-9), OPC-14523 or VPI-013 (see Bermack et al. "Effects of the potential antidepressant OPC-14523 [1-[3-[4-(3-chlorophenyl)-1-piperazinyl]propyl]-5-methoxy-3,4-dihydro-2-quinolinone monomethanesulfonate] a combined sigma and 5-HT1 A ligand: modulation of neuronal activity in the dorsal raphe nucleus." J PharmacolExp Ther. 2004 310(2):578-83), Casopitant or GW679769 (CAS RN 852393-14-7), Elzasonan or CP- 448,187 (CAS RN 361343-19-3), GW823296 (see published U.S. Patent Application US2005/0119248), Delucemine or NPS 1506 (CAS RN 186495-49-8), or Ocinaplon (CAS RN 96604-21-6).

Yet additional examples of such agents include CX717 from Cortex Pharmaceuticals, TGBAOLAD (a serotonin reuptake inhibitor, 5-HT2 agonist, 5-HT1A agonist, and 5-HT1D agonist) from Fabre-Kramer Pharmaceuticals, Inc., ORG 4420 (an NaSSA (noradrenergic/specific serotonergic antidepressant) from Organon, CP-316,311 (a CRF1 antagonist) from Pfizer, BMS-562086 (a CRF1 antagonist) from Bristol-Myers Squibb, GW876008 (a CRF1 antagonist) from Neurocrine/GlaxoSmithKline, ONO-2333Ms (a CRF1 antagonist) from Ono Pharmaceutical Co., Ltd., JNJ-19567470 or TS-041 (a CRF1 antagonist) from Janssen (Johnson & Johnson) and Taisho, SSR 125543 or SSR 126374 (a CRF1 antagonist) from Sanofi-Aventis, Lu AA21004 and Lu AA24530 (both from H. Lundbeck AJS), SEP-225289 from Sepracor Inc., ND7001 (a PDE2 inhibitor) from Neuro3d, SSR 411298 or SSR 101010 (a fatty acid amide hydrolase, or FAAH, inhibitor) from Sanofi- Aventis, 163090 (a mixed serotonin receptor inhibitor) from GlaxoSmithKline, SSR 241586 (an NK2 and NK3 receptor antagonist) from Sanofi-Aventis, SAR 102279 (an NK2 receptor antagonist) from Sanofi-Aventis, YKP581 from SK Pharmaceuticals (Johnson & Johnson), R1 576 (a GPCR modulator) from Roche, or ND 1251 (a PDE4 inhibitor) from Neuro3d.

In other embodiments described herein, a method may comprise use of a combination of a nootropic agent and one or more agents reported as anti-psychotic agents. Non-limiting examples of a reported anti-psychotic agent as a member of a combination include olanzapine, quetiapine (Seroquel), clozapine (CAS RN 5786-21-0) or its metabolite ACP-104 (N-desmethylclozapine or norclozapine, CAS RN 6104-71-8), reserpine, aripiprazole, risperidone, ziprasidone, sertindole, trazodone, paliperidone (CAS RN 144598-75-4), mifepristone (CAS RN 84371-65-3), bifeprunox or DU-127090 (CAS RN 350992-10-8), asenapine or ORG 5222 (CAS RN 65576-45-6), iloperidone (CAS RN 133454-47-4), ocaperidone (CAS RN 129029-23-8), SLV 308 (CAS RN 269718-83-4), licarbazepine or GP 47779 (CAS RN 29331-92-8), Org 34517 (CAS RN 189035-07-2), ORG 34850 (CAS RN 162607-84-3), Org 24448 (CAS RN 211735-76-1), lurasidone (CAS RN 367514-87-2), blonanserin or lonasen (CAS RN 132810-10-7), Talnetant or SB-223412 (CAS RN 174636- 32-9), secretin (CAS RN 1393-25-5) or human secretin (CAS RN 108153-74-8) which are endogenous pancreatic hormones, ABT 089 (CAS RN 161417-03-4), SSR 504734 (see compound 13 in Hashimoto "Glycine Transporter Inhibitors as Therapeutic Agents for Schizophrenia." Recent Patents on CNS Drug Discovery, 2006 1 :43-53), MEM 3454 (see Mazurov et al. "Selective alpha7 nicotinic acetylcholine receptor ligands." Curr Med Chem. 2006 13(13): 1567-84), a phosphodiesterase 10A (PDEIOA) inhibitor such as papaverine (CAS RN 58-74-2) or papaverine hydrochloride (CAS RN 61-25-6), paliperidone (CAS RN 144598-75-4), trifluoperazine (CAS RN 117-89-5), or trifluoperazine hydrochloride (CAS RN 440-17-5). Additional non-limiting examples of such agents include trifluoperazine, fluphenazine, chlorpromazine, perphenazine, thioridazine, haloperidol, loxapine, mesoridazine, molindone, pimoxide, or thiothixene, SSR 146977 (see Emonds-Alt et al. "Biochemical and pharmacological activities of SSR 146977, a new potent nonpeptide tachykinin NK3 receptor antagonist." Can J Physiol Pharmacol. 2002 80(5):482-8), SSR181507 ((3-exo)-8-benzoyl-N-[[(2 s)7-chloro-2,3-dihydro-1,4-benzodioxin-1-yl]methyl]-8-azabicyclo[3.2.1]octane-3-methanamine monohydrochloride), or SLV313 (1-(2,3-dihydro- benzo [1,4] dioxin-5-yl)-4- [5 -(4-fluorophenyl)-pyridin-3 -ylmethyl] -piperazine).

Further examples of such agents include Lu-35-138 (a D4/5-HT antagonist) from Lundbeck, AVE 1625 (a CB1 antagonist) from Sanofi-Aventis, SLV 310,313 (a 5-HT2 A antagonist) from Solvay, SSR 181507 (a D2/5-HT2 antagonist) from Sanofi-Aventis, GW07034 (a 5-HT6 antagonist) or GW773812 (a D2, 5-HT antagonist) from GlaxoSmithKline, YKP 1538 from SK Pharmaceuticals, SSR 125047 (a sigma receptor antagonist) from Sanofi-Aventis, MEM 1003 (a L-type calcium channel modulator) from Memory Pharmaceuticals, JNJ-17305600 (a GLYT1 inhibitor) from Johnson & Johnson, XY 2401 (a glycine site specific NMDA modulator) from Xytis, PNU 170413 from Pfizer, RGH- 188 (a D2, D3 antagonist) from Forrest, SSR 180711 (an alpha7 nicotinic acetylcholine receptor partial agonist) or SSR 103800 (a GLYT1 (Type 1 glycine transporter) inhibitor) or SSR 241586 (a NK3 antagonist) from Sanofi-Aventis. In other disclosed embodiments, a reported anti-psychotic agent may be one used in treating schizophrenia. Non-limiting examples of a reported anti-schizophrenia agent as a member of a combination with a nootropic agent include molindone hydrochloride (MOB AN®) and TC- 1827 (see Bohme et al. "In vitro and in vivo characterization of TC- 1827, a novel brain α4β2 nicotinic receptor agonist with pro-cognitive activity." Drug Development Research 2004 62(1):26-40).

### Agonists/Antagonists/Partial Agonists

In some embodiments described herein, Meparfynol, or a pharmaceutically acceptable salt thereof is used in combination with an additional agent which has "selective" activity (such as in the case of an antagonist or inverse agonist) under certain conditions against one or more opioid receptor subtypes with respect to the degree and/or nature of activity against one or more other opioid receptor subtypes. For example, in some embodiments described herein, the additional agent has an antagonist effect against one or more subtypes, and a much weaker effect or substantially no effect against other subtypes. As another example, an additional agent used in the methods described herein may act as an agonist at one or more opioid receptor subtypes and as antagonist at one or more other opioid receptor subtypes. In some embodiments described herein, the additional agent has activity against kappa opioid receptors, while having substantially lesser activity against one or both of the delta and mu receptor subtypes. In other embodiments described herein, the additional agent has activity against two opioid receptor subtypes, such as the kappa and delta subtypes. As examples, the agents naloxone and naltrexone have nonselective antagonist activities against more than one opioid receptor subtypes. In certain embodiments described herein, selective activity of one or more opioid antagonists results in enhanced efficacy, fewer side effects, lower effective dosages, less frequent dosing, or other desirable attributes. An opioid receptor antagonist is an agent able to inhibit one or more characteristic responses of an opioid receptor or receptor subtype. As an example, an antagonist may competitively or non-competitively bind to an opioid receptor, an agonist or partial agonist (or other ligand) of a receptor, and/or a downstream signaling molecule to inhibit a receptor's function.

An inverse agonist able to block or inhibit a constitutive activity of an opioid receptor may also be used. An inverse agonist may competitively or non-competitively bind to an opioid receptor and/or a downstream signaling molecule to inhibit a receptor's function. Examples of inverse agonists for use in the disclosed methods include an NMDA receptor agonist/antagonist, an AMPA receptor agonist/antagonist, ICI-174864 (N,iV-diallyl-Tyr-Aib-Aib-Phe-Leu), RTI-5989-1, RTI-5989-23, and RTI-5989-25 (see Zaki et al. J. Pharmacol. Exp. Therap. 298(3): 1015-1020, 2001).

### 5HT3/5HT2 receptor antagonists

Additionally, Meparfynol, or a pharmaceutically acceptable salt thereof may be used with a reported 5HT3 receptor antagonist such as azasetron (CAS RN 123039-99-6); Ondansetron (CAS RN 99614-02-5) or Ondansetron hydrochloride (CAS RN 99614-01-4); Cilansetron (CAS RN 120635-74-7); Aloxi or Palonosetron Hydrochloride (CAS RN 135729-62-3); Palenosetron (CAS RN 135729-61-2 or 135729-56-5); Cisplatin (CAS RN 15663-27-1); Lotronex or Alosetron hydrochloride (CAS RN 122852-69-1); Anzemet or Dolasetron mesylate (CAS RN 115956-13-3); zacopride or R-Zacopride; E-3620 ([3(S)-endo]-4-amino-5-chloro-N-(8- methyl- 8-azabicyclo[3.2.1-]oct-3-yl-2[(1-methyl-2-butynyl)oxy]benzamide) or E-3620 HCl (3(S)-endo-4-amino-5-chloro-N-(8-methyl-8-azabicyclo [3.2.1] oct-3-yl)-2-(1-methyl-2- butinyl)oxy)-benzamide-HCl); YM 060 or Ramosetron hydrochloride (CAS RN 132907-72- 3); athieno[2,3-d]pyrimidine derivative antagonist described in U.S. Patent 6,846,823, such as DDP 225 or MCI 225 (CAS RN 135991-48-9); Marinol or Dronabinol (CAS RN 1972-08- 3); or Lac Hydrin or Ammonium lactate (CAS RN 515-98-0); Kytril or Granisetron hydrochloride (CAS RN 107007-99-8); Bemesetron (CAS RN 40796-97-2); Tropisetron (CAS RN 89565-68-4); Zatosetron (CAS RN 123482-22-4); Mirisetron (CAS RN 135905- 89-4) or Mirisetron maleate (CAS RN 148611-75-0); or renzapride (CAS RN 112727-80-7).

Additionally, Meparfynol, or a pharmaceutically acceptable salt thereof may be used with a reported 5HT2A/2C receptor antagonist such as Ketanserin (CAS RN 74050-98-9) or ketanserin tartrate; risperidone; olanzapine; adatanserin (CAS RN 127266-56-2); Ritanserin (CAS RN 87051-43-2); etoperidone; nefazodone; deramciclane (CAS RN 120444-71-5); Geoden or Ziprasidone hydrochloride (CAS RN 138982-67-9); Zeldox or Ziprasidone or Ziprasidone hydrochloride; EMD 281014 (7-[4-[2-(4-fluoro-phenyl)-ethyl]-piperazine-1-carbonyl]-1H- indole-3-carbonitrile HCl); MDL 100907 or M100907 (CAS RN 139290-65-6); Effexor XR (Venlafaxine formulation); Zomaril or Iloperidone; quetiapine (CAS RN 111974-69-7) or Quetiapine fumarate (CAS RN 111974-72-2) or Seroquel; SB 228357 or SB 243213 (see Bromidge et al. "Biarylcarbamoylindolines are novel and selective 5-HT(2C) receptor inverse agonists: identification of 5-methyl-1-[[2-[(2-methyl-3-pyridyl)oxy]- 5- pyridyl] carbamoyl] -6-trifluoromethylindoline (SB-243213) as a potential antidepressant/anxiolytic agent." J Med Chem. 2000 43(6): 1123-34; SB 220453 or Tonabersat (CAS RN 175013-84-0); Sertindole (CAS RN 106516-24-9); Eplivanserin (CAS RN 130579-75-8) or Eplivanserin fumarate (CAS RN 130580-02-8); Lubazodone hydrochloride (CAS RN 161178-10-5); Cyproheptadine (CAS RN 129-03-3); Pizotyline or pizotifen (CAS RN 15574-96-6); Mesulergine (CAS RN 64795-35-3); Irindalone (CAS RN 96478-43-2); MDL 11939 (CAS RN 107703-78-6); or pruvanserin (CAS RN 443144-26-1).

Additional examples of modulators include reported 5-HT2C agonists or partial agonists, such as m-chlorophenylpiperazine; or 5-HT2A receptor inverse agonists, such as ACP 103 (CAS RN: 868855-07-6), APD125 (from Arena Pharmaceuticals), AVE 8488 (from Sanofi-Aventis) or TGWOOAD/AA(from Fabre Kramer Pharmaceuticals).

In yet further embodiments described herein, Meparfynol, or a pharmaceutically acceptable salt thereof is used in combination with an additional agent which has 5-HT6 receptor antagonist activity. In this regard, a 5-HT6 antagonist is selective for 5-HT6 receptors. By way of example, the the 5-HT6 antagonist may have a greater than 10-fold selectivity for this receptor over other binding sites within the CNS, in particular other 5-HT receptor sub-types and dopaminergic receptors. The 5-HT6 antagonists demonstrate greater than 100-fold selectivity for 5-HT6 receptors. The selectivity of the antagonist for 5-HT6 receptors can be determined using binding assays methods which are well known to those skilled in the art.

Preferred 5-HT6 antagonists include those disclosed in patent applications WO 98/27081 (SmithKline Beecham plc) and WO 99/02502 (SmithKline Beecham plc). Antagonists disclosed in WO 2000/012073 include compounds of formula (A) and compounds of formula (B), which can be prepared according to methods described in WO 98/27081 and WO 99/02502 respectively. Other antagonists disclosed in WO 2000/012073 include those generically and specifically disclosed in patent application WO 97/27058 (SmithKline Beecham) and European patent applications EP 0815861 (Hoffman-la-Roche) and EP 0930302 (Hoffman-la-Roche).

Particularly preferred antagonists disclosed in WO 2000/012073 include 5-Chloro-3-methylbenzobthiophene-2-sulfonic acid (4-methoxy-3-piperazin-1-ylphenyl) amide (Example 83 in WO 98/27081), that is to say, the compound of formula (I) and N-(2,5-Dibromo-3-fluorophenyl)-4-methoxy-3-piperazin-1-ylbenzenesulfonamide (Example 140 in WO 99/02502) that is to say, the compound of formula (II). Compounds exhibiting 5-HT6 receptor antagonist activity may form acid addition salts with acids, such as conventional pharmaceutically acceptable acids, for example maleic, hydrochloric, hydrobromic, phosphoric, acetic, fumaric, salicylic, sulphate, citric, lactic, mandelic, tartaric and methanesulphonic. Salts of 5-HT6 receptor antagonists therefore form an aspect of the present disclosure. Suitably, a compound of formula (I) and (II) are used as the hydrochloride salt.

SmithKline Beecham is developing the 5-HT6 antagonist, SB-271046, as a potential cognition enhancer. By December 1999, phase I trials had commenced. This drug was originally being developed primarily for the treatment of schizophrenia, however, cognitive disorders, including but not limited to Alzheimer's disease, have been the main target since 1998. SB-271046 is a potent, selective 5-HT6 antagonist with a pKi value of 8.9. SB-258585, also known as 4-iodo-N-[4-methoxy-3-(4-methylpiperazin-1-yl)phenyl]benzenesulfonamide is an analog of SB-271046. Data presented at the Society for Neuroscience annual meeting in November 2000 demonstrated that administration of SB-271046 resulted in a signficant increase in glutamate and aspartate levels in the frontal cortex, without affecting noradrenaline, dopamine or 5-HT levels. This was stated to suggest that 5-HT6 antagonists might therefore be useful for treating cognitive dysfunction. The drug has also been radiolabeled in order to provide an assay for *estimating in vivo* 5-HT6 receptor occupancy.

### Anti-epileptic agents

In some embodiments described herein, Meparfynol, or a pharmaceutically acceptable salt thereof is used in combination with an anti-epileptic agent. In a related embodiment described herein, the anti-epileptic agent is selected from the group consisting of valproate, divalproex, gabapentin, topiramate, leviracetam, lamotrigine, carbamazapine, oxcarbamazepine, tiagabine, zonisamide, clonzaepam, pregabalin, zarontin and mixtures thereof. In another embodiment described herein, Meparfynol or a pharmaceutically acceptable salt thereof is used in combination with a stimulant or non-stimulant of attention. In a related embodiment described herein, the stimulant or non-stimulant of attention is selected from the group consisting of dextroamphetamine, methylphenidate, adderall, adderall XR, concerta, focalin, and strattera.

### Neuroleptic agents and/or Neuropsychiatric agents

In some embodiments described herein, Meparfynol or a pharmaceutically acceptable salt thereof is administered in combination with one or more additional therapeutic agents. The additional therapeutic agents can include an antipsychotic agent, such as, but not limited to neuroleptic agents and/or a neuropsychiatric agents.

As there are currently no cures for schizophrenia, the objective of treatment is to reduce the severity of the symptoms, if possible to the point of remission. Due to the similarities in symptoms, schizophrenia and bipolar disorder are often treated with some of the same medicaments. Both diseases are often treated with antipsychotics such as neuroleptic agents and/or a neuropsychiatric agents.

Neuroleptic drugs include phenothiazines including aliphatics (e.g., chlorpromazine), piperidines (e.g., thioridazine), and piperazines (e.g., fluphenazine); butyrophenones (e.g., haloperidol); thioxanthenes (e.g., flupenthixol); oxoindoles (e.g., molindone); dibenzoxazepines (e.g., loxapine) and diphenylpiperidines (e.g., pimozide). Unfortunately, neuroleptics-resistant negative symptoms account for most of the social and vocational disability caused by schizophrenia. Further, neuroleptics cause extrapyramidal symptoms, including rigidity, tremor, bradykinesia (slow movement), and bradyphrenia (slow thought), as well as tardive dyskinesias and dystonias (involuntary muscular contractions). For treatment of psychosis with medications, see, Textbook of Psychopharmacology, Schatzberg A F and Nemeroff C B, Editors, American Psychiatric Press. Wash.D.C.1995.

As used herein, a "neuropsychiatric agent" refers to a compound, or a combination of compounds, that affects the neurons in the brain either directly or indirectly and/or competitively and/or non-competitively (such as, for example, working at a site which is distal to receptor rather than at a receptor site itself), or affects the signal transmitted to the neurons in the brain. Neuropsychiatric agents, therefore, may affect a person's psyche, such as the person's mood, perception, nociception, cognition, alertness, memory and the like. In certain embodiments, the neuropsychiatry agent may be selected from the group consisting of monoamine reuptake inhibitiors, selective serotonin reuptake inhibitors, norepinephrine reuptake inhibitors, dual serotonin and norepinephrine reupake inhibitors, dopamine agonists, antipsychotic agents, inverse serotonin agonists, serotonin antagonists, serotonin 2 inverse agonists, serotonin 2 antagonists, serotonin IA agonists, anti epileptic and peripherally acting muscarinic antagonists.

### Atypical Antipsychotics

In yet another embodiment described herein, the aforementioned method is provided further comprising administering an atypical antipsychotic. In a related embodiment described herein, the atypical antipsychotic is selected from the group consisting of risperidone, olanzepine, quetiapine, ziprasidone, and aripiprazole. In still another embodiment described herein, the aforementioned method is provided further comprising administering a cholinergic enhancer. In a related embodiment described herein, the cholinergic enhancer is selected from the group consisting of Aricept (donepezil), excelon, reminyl (galantamine), and mestinon.

Antipsychotic drugs, in one form or another, have long been the basis of treatment of psychotic disorders. These drugs are sometimes used in combination with a mood regulating medication such as lithium or an antidepressant. As used herein, the term "antipsychotic drug" comprises a medication used to treat schizophrenia and psychotic symptoms (such as hallucinations, delusions and other positive symptoms as defined in DSM-IV) in other psychiatric illnesses, and further comprises both typical and atypical antipsychotic drugs.

As used herein, the term "typical antipsychotic drug" encompasses chlorpromazine (Thorazine), fluphenazine (Prolixin), haloperidol (Haldol), thiothixene (Navane), trifluoperazine (Stelazine), perphenazine (Trilafon), and thioridazine (Mellaril).

For symptoms associated with schizophrenia, antipsychotic medications are the most common and most valuable treatments. Up until fairly recently, the attention of researchers working in the field of the biochemistry of psychoses was mainly concentrated on two mediator systems: the dopamine system and the serotonin system. Abnormal activity of the neurotransmitter dopamine is a hallmark of schizophrenia. Dopaminergic activity is reduced in the mesocortical system (resulting in negative symptoms) and is enhanced in the mesolimbic system (resulting in positive or psychotic symptoms). Several other neurotransmitters are involved, including serotonin, glutamate, and gamma-aminobutyric acid (GABA). More recently, the importance of the glutamate system in the pathophysiology of schizophrenia has been highlighted (see, for example, Patil et al (2007) Nature Medicine 7: 1102-1107 "Activation of mGlu2/3 receptors as anew approach to treat schizophrenia: a randomized Phase 2 clinical trial".

In accordance with the dopamine theory of schizophrenia, for many years, schizophrenia was treated with classical antipsychotic drugs, the neuroleptics that block central dopamine receptors. The neuroleptics are effective for treating the positive symptoms (hallucinations, delusions, and conceptual disorganisation) of schizophrenia, but have little or no effect on the negative symptoms (apathy, social withdrawal, affect, and poverty of speech). The ability of these drugs to antagonize dopamine receptors correlates with antipsychotic efficacy.

However, more recently, publication of research by ACADIA in the Journal of Pharmacology and Experimental Therapeutics (JPET) has shown that ACP-104, the major metabolite of clozapine, is a partial agonist that causes weak activation of dopamine D2 and D3 receptors, whereas clozapine and most other antipsychotic drugs block these receptors. ACADIA believes that these partial agonist properties of ACP-104 may lead to less motoric side effects than seen with most other antipsychotic drugs. ACADIA is developing ACP-104 as a novel therapy for schizophrenia, with the added potential benefit of improving cognition.

The JPET article (August 31, 2005, e-pub) describes the research conducted by ACADIA scientists of 41 marketed and experimental antipsychotic drugs and their ability to alter the activities of dopamine D2, D3, and D4 receptors. Using ACADIA's proprietary R-SAT(R) technology platform, the scientists developed assays that detect subtle changes in the activities of these receptors. Among the 41 drugs, only two of them, ACP-104 and aripiprazole (marketed as ABILIFY(R)), were partial agonists causing weak activation of dopamine D2 and D3 receptors. Most antipsychotic drugs were shown to block these dopamine receptors. Prior research has shown that blockade of the dopamine receptors may cause undesirable motoric side effects, including Parkinson-like symptoms and tardive dyskinesia.

ACP-104 is a small molecule drug candidate that ACADIA has discovered and is developing as a novel, stand-alone therapy for schizophrenia. It combines an atypical antipsychotic efficacy profile with the added potential benefit of improving cognition, one of the major challenges in treating schizophrenia today. ACP-104, or N-desmethylclozapine, is the major metabolite of clozapine, and has a unique ability to stimulate M1 muscarinic receptors. The M1 muscarinic receptors are widely known to play an important role in cognition. ACADIA is currently conducting initial Phase II studies to evaluate safety, tolerability, and preliminary efficacy of ACP-104 in patients with schizophrenia.

Thus, in the context of the treatment of schizophrenia in a subject in need of such treatment the term "Combination therapy" (or "co-therapy") includes the administration of Meparfynol and at least a second agent, for example: dopamine agonists such as bromocriptine, cabergoline, lisuride, pergolide, ropinirole, amantadine, apomorphine, sumanirole, rotigotine, talipexole, dihydroergocriptine and pramipexole, levodopa, levodopa plus carbidopa (SINEMET®), levodopa plus controlled release carbidopa (SINEMET-CR®), levodopa plus benserazide (MADOPAR®), levodopa plus controlled release benserazide (MADOPAR-HBS), COMT inhibitors such as tolcapone and entacapone, - STALEVO®, Amantadine, ACP-104, N-desmethylclozapine, carmoxirole, optically pure-didesmethylsibutramine, dopexamine, fenoldopam, ibopamine, lergotrile, lisuride, memantine, mesulergine, pergolide, piribedil, pramipexole, quinagolide, ropinirole, roxindole, and talipexole or a pharmaceutically acceptable salt, solvate, or hydrate of any of them as part of a specific treatment regimen intended to provide the beneficial effect by counteracting the negative effects on cognition of the above mentioned agents (alone or in different combination) and at the same time further ameliorate unwanted side effects, such as, for example, motor dysfunction in certain disease states.

More recently, progress in the treatment of psychotic conditions has been achieved through the introduction of new, atypical antipsychotic agents. The side effect profile of these atypical antipsychotics is far superior to that of traditional agents. The atypical antipsychotics are a different class of antipsychotic drugs which have a different receptor binding profile and effectiveness against the symptoms of schizophrenia. The essential feature of an atypical antipsychotic is less acute extrapyramidal symptoms (such as rigidity, tremor), especially dystonias (such as involuntary muscular contractions), associated with therapy as compared to a typical antipsychotic such as haloperidol. Atypical antipsychotics bind central serotonin 2 (5-HT2) receptors in addition to D2 dopamine receptors. Unlike the neuroleptics, they improve negative as well as positive symptoms. They cause minimal extrapyramidal symptoms and rarely cause tardive dyskinesias, akathisia, or acute dystonic reactions.

As used herein, the term "atypical antipsychotic drug" encompasses aripiprazole (Ability), risperidone (Risperdal), clozapine (Clozaril), olanzapine (Zyprexa), quetiapine (Seroquel), sertindole (Serdolect) and ziprasidone (Geodon).

The first atypical antipsychotic drug approved for the treatment of schizophrenia was clozapine. Clozapine, the prototypical atypical antipsychotic, is effective for the treatment of schizophrenia, especially for subjects who do not respond to traditional neuroleptic therapy. Clozapine, differs from the typical antipsychotics with the following characteristics: (1) greater efficacy in the treatment of overall psychopathology in patients with schizophrenia nonresponsive to typical antipsychotics; (2) greater efficacy in the treatment of negative symptoms of schizophrenia; and (3) less frequent and quantitatively smaller increases in serum prolactin concentrations associated with therapy (Beasley, et al., Neuropsychopharmacology, 14(2), 111-123, (1996)).

Clozapine, 8-chloro-11-(4-methyl-1-piperazinyl)-5H-dibenzo[b,e][1,4]diazepine, is described in U.S. Patent No. 3,539,573. Clinical efficacy in the treatment of schizophrenia is described (Hanes, et al., Psychopharmacol. Bull 24, 62 (1988)).

Olanzapine, 2-methyl-4-(4-methyl-1-piperazinyl)-10H-thieno[2,3 b][1,5]benzodiazepine, is a known compound and is described in U.S. Pat. No. 5,229,382 as being useful for the treatment of schizophrenia, schizophreniform disorder, acute mania, mild anxiety states, and psychosis.

Risperidone,3-[2-[4-(6-fluoro-1,2-benzisoxazol-3-yl)piperidino]ethyl]-2-methyl-6,7,8,-9 tetrahydro-4H-pyrido-[1,2-a]pyrimidin-4-one, and its use in the treatment of psychotic diseases are described in U.S. Pat. No. 4,804,663.

Sertindole,1-[2-[4-[5-chloro-1-(4-fluorophenyl)-1H-indol-3-yl]-1-piperidinyl]ethyl]I midazolidin-2-one, is described in U.S. Pat. No. 4,710,500. Its use in the treatment of schizophrenia is described in U.S. Pat. Nos. 5,112,838 and 5,238,945.

Quetiapine, 5-[2-(4-dibenzo[b,f][1,4]thiazepin-11-yl-1-piperazinyl)ethoxy-]ethanol, and its activity in assays which demonstrate utility in the treatment of schizophrenia are described in U.S. Pat. No. 4,879,288. Quetiapine is typically administered as its (E)-2-butenedioate (2:1) salt.

Ziprasidone, 5-[2-[4-(1,2-benzoisothiazol-3-yl)-1-piperazinyl]ethyl]-6-chloro-1,3-dihydro-2H-indol-2-one, is typically administered as the hydrochloride monohydrate. The compound is described in U.S. Pat. Nos. 4,831,031 and 5,312,925. Its activity in assays which demonstrate utility in the treatment of schizophrenia are described in U.S. Pat. No. 4,831,031. U.S. Pat. Nos. 4,831,031 and 5,312,925.

In one preferred embodiment described herein, the term "combination therapy" refers to the treatment of a human subject with Meparfynol, or a pharmaceutically acceptable salt thereof and one or more other antipsychotic drugs, preferably an atypical antipsychotic drug such as aripiprazole, risperidone, clozapine, olanzapine, quetiapine or ziprasidone.

In some embodiments of combination administration described herein, Meparfynol, or a pharmaceutically acceptable salt thereof is administered in combination with another therapeutic agent, wherein at least a portion of Meparfynol, or a pharmaceutically acceptable salt thereof is administered by directly introducing the Meparfynol, or a pharmaceutically acceptable salt thereof to a subject.

Thus, for example, clozapine may be administered in combination with Meparfynol, or a pharmaceutically acceptable salt thereof wherein both clozapine and Meparfynol, or a pharmaceutically acceptable salt thereof are directly administered to a subject. In one embodiment described herein, directly introducing Meparfynol, or a pharmaceutically acceptable salt thereof to a subject may be accomplished by the subject orally ingesting Meparfynol. In one embodiment described herein, directly introducing Meparfynol, or a pharmaceutically acceptable salt thereof to a subject may be accomplished by intravenously injecting Meparfynol into the subject.

Preferred compounds which can be used as monotherapy, or in combination with Meparfynol or pharmaceutically acceptable salts thereof in an effective amount for treating one or more cognitive disorder in a patient, are acetylcholine cholinesterase inhibitors such as but not limited to Donepezil hydrochloride, or Aricept® which are disclosed and described in US Patent No. 4895841, WO2001/066114 and EP1311272B1 and/or a selective metabotropic glutamate receptor agonist, such as a selective agonist for metabotropic gluatamate 2/3 (mGlu2/3) receptors (such as those (Eli Lilly LY404039 and LY2140023) disclosed in Nature Reviews Drug Discovery (Dec 2007) 6: DO110.1038/nrd2452).

In another preferred embodiment described herein, the term "combination therapy" refers to the treatment of a human subject with Meparfynol, or a pharmaceutically acceptable salt thereof and one or more other cholinesterase inhibitor drugs, such as Donepezil hydrochloride, or Aricept® which is disclosed and described in US Patent No4895841, WO2001/066114 and EP1311272B1.

In yet another preferred embodiment described herein, the term "combination therapy" refers to the treatment of a human subject with Meparfynol, or a pharmaceutically acceptable salt thereof and one or more other selective metabotropic glutamate receptor agonist, such as a selective agonist for metabotropic gluatamate 2/3 (mGlu2/3) receptors (such as those (Eli Lilly LY404039 and LY2140023) disclosed in Nature Reviews Drug Discovery (Dec 2007) 6: DO110.1038/nrd2452).

In a further preferred embodiment described herein, the term "combination therapy" refers to the treatment of a human subject with Meparfynol, or a pharmaceutically acceptable salt thereof and one or more of a 5HT-4 selective agonist and/or an acetylcholine cholinesterase inhibitors such as but not limited to Donepezil hydrochloride, or Aricept® which are disclosed and described in US Patent No. 4895841, WO2001/066114 and EP1311272B1.

Preferred combinations of (1) Meparfynol, or a pharmaceutically acceptable salt thereof and (2) an additional agent selected from the group consisting of: (i) an NMDA/AMPA receptor modulator; (ii) a nootropic agent; (iii) an anti-depressive agent (eg SSRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant as described herein are "synergistic", meaning that the therapeutic effect of co- administering compounds selected from (1) and (2) as defined above is greater than additive. Thus, co-administering both therapeutic agents produces an effect which is greater than the sum of the effects of each agent administered alone. Such synergy is advantageous in that it allows for each therapeutic agent typically to be administered in an amount less than if the combined therapeutic effects were additive. Thus, therapy can be effected for subjects who, for example, do not respond adequately to the use of one component at what would be considered a maximal strength dose. Additionally, by administering the components in lower amounts relative to the case where the combined effects are additive, side effects such as those described above can be minimized or avoided in many cases. Such synergy can be demonstrated by the tests disclosed and described below.

The synergy of such preferred combinations is provided as a further feature of the present disclosure, and accordingly the disclosure provides a method for achieving a synergistic therapeutically effective level of treatment of schizophrenia in a subject in need of such treatment comprising co-administering to a mammal in need of such treatment (1) Meparfynol, or a pharmaceutically acceptable salt thereof and (2) an additional agent selected from the group consisting of: (i) an NMDA/AMPA receptor modulator; (ii) a nootropic agent; (iii) an anti-depressive agent (eg an SSRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant wherein the amount of the first compound alone and the amount of the second agent alone are each insufficient to achieve the synergistic therapeutically effective level of behavioural response treatment and/or pharmacological functional activity (such as, for example, enhanced sensory processing function and/or enhanced cognitive function), but wherein the combined effect of the amounts of the first and second compounds is greater than the sum of the levels of therapeutic effects of behavioural response treatment and/or pharmacological functional activity, achievable with the individual amounts of the first compound and second agent.

Additional preferred combinations include those which can be taken "on demand", as opposed to needing to be taken chronically.

Additional preferred combinations include those which are "fast acting", meaning that the time taken from administration to the point at which the behavioural response can be modulated is less than about two hours, preferably less than about one hour, more preferably on the order of a half hour or less, and even more preferably within 10 or 15 minutes.

As used herein, the term "Combination therapy" is intended to embrace administration of these therapeutic agents and/or treatments in a separate, sequential manner or simultaneously. In this regard, the term "combination therapy" can cover the co-administration of more than one compound separately but as part of the same therapeutic treatment program or regimen, and it is contemplated that separate administration of each compound, at different times and by different routes, will sometimes be recommended. Thus, two or more compounds need not necessarily be administered at essentially the same time. In a preferred embodiment, administration is timed so that the peak pharmacokinetic effect of one compound coincides with the peak pharmacokinetic effect for the other. If co-administered separately, it is also preferred, so that, for example, two compounds may be administered in an oral dosage form.

Simultaneous administration can be accomplished, for example, by administering to the subject a single capsule having a fixed ratio of each therapeutic agent or in multiple, single capsules for each of the therapeutic agents. In some embodiments described herein, Meparfynol, or a pharmaceutically acceptable salt thereof and an additional therapeutic agent(s) are administered nearly simultaneously. These embodiments include those in which the compounds are in the same administrable composition, that is, a single tablet, pill, or capsule, or a single solution for intravenous injection, or a single drinkable solution, or a single dragee formulation or patch, which contains the compounds. The embodiments also include those in which each compound is in a separate administrable composition, but the patient is directed to take the separate compositions nearly simultaneously, such as, for example, when one pill is taken right after the other or that one injection of one compound is made right after the injection of another compound.

Sequential or substantially simultaneous administration of each therapeutic agent can be effected by any appropriate route including oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Sequential administration can also be achieved, for example, using a single dosage form, for example a dosage form such as an oral tablet that has two different layers with different release profiles for the two active ingredients. One of ordinary skill in the art will appreciate that various other forms of administration and application patterns are conceivable within the context of the present disclosure.

As most of the compounds disclosed herein are orally available and are normally administered orally, oral administration of any drug combination is preferred. In this regard, the compounds may be administered together, in a single dosage form, or may be administered separately. However, oral administration is not the only route or even the only preferred route. For example, transdermal administration may be very desirable for patients who are forgetful or petulant about taking oral medicine. In this regard, any one of the compounds disclosed herein may be administered by one route, such as oral, and the other(s) may be administered by the transdermal, percutaneous, intravenous, intramuscular, intranasal or intrarectal route, in particular circumstances. The route of administration may be varied in any way, limited by the physical properties of the drugs and the convenience of the patient and the caregiver.

In other embodiments described herein, Meparfynol, or a pharmaceutically acceptable salt thereof and an additional therapeutic compound is administered first and then the other one Meparfynol, or a pharmaceutically acceptable salt thereof and the additional therapeutic compound is administered second. In these embodiments described herein, the patient may be administered a composition comprising one of the compounds and then at some time, a few minutes or a few hours later, be administered another composition comprising the other one of the compounds. Also included in these embodiments are those in which the patient is administered a composition comprising one of the compounds on a routine or continuous basis while receiving a composition comprising the other compound occasionally.

In some instances, the term "combination therapy" may encompass the administration of two or more therapeutic compounds as part of separate monotherapy regimens that incidentally and arbitrarily result in the combinations contemplated by the present invention.

Administration of these therapeutic compounds in combination typically is carried out over a defined time period (usually minutes, hours, days or weeks depending upon the combination selected).

Alternatively, for example, all therapeutic compounds may be administered orally or all therapeutic agents may be administered by intravenous injection. The sequence in which the therapeutic agents are administered is not narrowly critical.

The term "Combination therapy" also can embrace the administration of the therapeutic compounds as described above in further combination with other biologically active ingredients and non-drug therapies (such as, for example, surgery or radiation treatment and/or behavioural response treatment or the like). Where the combination therapy further comprises a non-drug treatment, the non-drug treatment may be conducted at any suitable time so long as a beneficial effect from the combination of the therapeutic agents and non-drug treatment is achieved.

### PHARMACEUTICAL COMPOSITIONS

In one aspect, the invention includes Meparfynol or a pharmaceutically acceptable salt thereof according to the present invention administered as the active ingredient of a pharmaceutically acceptable composition, having activity in the treatment of schizophrenia in a subject in need of such treatment. This pharmaceutical composition can be prepared by conventional procedures, for instance by mixing the active agent with a pharmaceutically acceptable, therapeutically inert organic and/or inorganic carrier, diluents or excipient materials (including combinations thereof. The pharmaceutical compositions may be for human or animal usage in human and veterinary medicine and will typically comprise any one or more of a pharmaceutically acceptable diluent, carrier, or excipient.

The following present some non-limiting examples of formulations.

Formulation 1: A tablet is prepared using the following ingredients:

| | Weight/mg |
|---|---|
| Agent | 250 |
| Cellulose, microcrystalline | 400 |
| Silicon dioxide, fumed | 10 |
| Stearic acid | 5 |
| Total | 665 |

The components are blended and compressed to form tablets each weighing 665mg. Formulation 2: An intravenous formulation may be prepared as follows:
Agent 100mg
Isotonic saline 1,000ml

### MODES OF ADMINISTRATION

The invention further provides Meparfynol or a pharmaceutically acceptable salt thereof for the prevention and/or treatment of schizophrenia in an individual, by for example, administering to an individual Meparfynol or a pharmaceutically acceptable salt thereof.

As used herein, the term "administered" includes delivery by a mucosal route, for example, as a nasal spray or aerosol for inhalation or as an ingestable solution such as by an oral route, or by a parenteral route where delivery is by an injectable form, such as, for example, by a rectal, ophthalmic (including intravitreal or intracameral), nasal, topical (including buccal and sublingual), intrauterine, vaginal or parenteral (including subcutaneous, intraperitoneal, intramuscular, intravenous, intradermal, intracranial, intratracheal, and epidural) transdermal, intraperitoneal, intracranial, intracerebroventricular, intracerebral, intravaginal, intrauterine, or parenteral (e.g., intravenous, intraspinal, intracavernosal, subcutaneous, transdermal or intramuscular) route.

### DOSAGE

The dosage of the Meparfynol or a pharmaceutically acceptable salt thereof will depend on the disease state or condition being treated and other clinical factors including subject age, weight, diet and condition and response of the individual and the route and time of administration of the compound. Depending upon the half-life of the compound in the particular subject, the agent can be administered from several times per day to once a week. It is to be understood that the present invention has application for both human and veterinary use. The methods described herein contemplate single as well as multiple administrations, given either simultaneously or over an extended period of time. By way of example, suitable treatment may be given 1 or 2 or 3 times daily, depending upon clearance rate.

Optimal therapeutically effective doses to be administered may be readily determined by those skilled in the art and will vary, basically, with the strength of the preparation, with the mode of administration and with the advancement of the pathological condition or the specific memory or cognition disorder treated.

The dosages below are exemplary of the average case. There can, of course, be individual instances where higher or lower dosage ranges are merited and such are within the scope of this invention. The skilled person will appreciate that, in the treatment of certain conditions the agent may be taken as a single dose as needed or desired.

For use herein, unless clearly indicated otherwise, the compounds may be administered to the individual by any available dosage form.

Preferably the pharmaceutical composition is administered as a once daily dosage form.

In one variation, the compound is administered to the individual as a conventional immediate release dosage form.

By way of example, in one embodiment described herein, the pharmaceutical composition of the present invention comprising Meparfynol or a pharmaceutically acceptable salt thereof can contain, per dosage unit form, such as, for example, a capsule, tablet, powder injection, teaspoonful, suppository and the like form, from about 20 to 7000 mg of the active ingredient.

In another embodiment described herein, amount of Meparfynol, in a delivery form may be any effective amount, which may be from about 10 mg to about 1500 mg or more.

In one variation, a delivery form, such as a sustained release system, may comprises less than about 30 mg of Meparfynol or a pharmaceutically acceptable salt thereof. In another variation, a delivery form, such as a single sustained release system capable of multi-day administration, comprises an amount of compound such that the daily dose of compound is less than about 30 mg of Meparfynol or a pharmaceutically acceptable salt thereof.

In another embodiment described herein, the desired dose may be presented in a single dose or as divided doses administered at appropriate intervals, for example, two to four or more sub-doses per day. By way of example, a useful intravenous dose is typically from about 5 and 50 mg and a useful oral dosage is typically from about 50 to 600 mg, preferably from about 20 and 200 mg.

In a further embodiment described herein, the subject in need of the above mentioned treatment is administered a dose of Meparfynol or a pharmaceutically acceptable salt thereof as above defined which ranges from about 0.3 to about 100 mg/kg of body weight per day.

In yet another embodiment described herein, Meparfynol or a pharmaceutically acceptable salt thereof is administered orally as a tablet or capsule comprising about 100 mg of Meparfynol. Thus, for example, about 100 mg capsules may be administered twice daily to achieve an about 200 mg per day dose. To achieve a 400 mg per day dose, two about 100 mg capsules may be administered twice daily (totaling 4 capsules over the day).

In some embodiments described herein, Meparfynol or a pharmaceutically acceptable salt thereof is administered orally in an amount totaling about 200 mg per day or about 400 mg per day. In some such embodiments described herein, Meparfynol or a pharmaceutically acceptable salt thereof is advantageously administered twice daily (e.g., about 100 mg twice daily or about 200 mg twice daily). While not being bound by any particular theory, twice daily administration is believed to attenuate fluctuations in blood levels and improve tolerability.

In yet further embodiments described herein, the dose which is administered to a subject is titrated to the final dose. For example, in some embodiments, a final dose of about 200 mg per day dose is achieved by first administering about 100 mg per day (e.g., about 50 mg twice daily) for a certain period of time followed by administering about 200 mg per day (e.g., about 100 mg twice daily). In some embodiments described herein, a final about 400 mg per day dose is achieved by first administering about 100 mg per day (e.g., about 50 mg twice daily) for a certain period of time, followed by administering about 200 mg per day (e.g., about 100 mg twice daily) for a certain period of time, followed by administering about 300 mg per day (e.g., about 150 mg twice daily) for a certain period of time, followed by administering the final about 400 mg per day (e.g., about 200 mg twice daily). Prior to each dose escalation, a physician may evaluate the patient to determine if a continued dose escalation is warranted. In some cases, the physician may decide to extend the amount of time that a lower dose is administered prior to escalation. In some embodiments described herein, the physician may decide to not increase the dosage any further, thereby choosing as a final dose a dose less than the originally planned final dose. In other embodiments described above, the desired administration doses may be achieved by administering a single capsule or tablet. Alternatively, the doses may be achieved by administering multiple capsules or tablets simultaneously or in sequence. In further embodiments described herein, all doses are achieved using tablets or capsules containing 50 mg or 100 mg of Meparfynol or a pharmaceutically acceptable salt thereof.

Preferably the maintenance dose of the pharmaceutical composition of Meparfynol, is a unit oral dosage form, which comprises from about 20 mg and 50 mg Meparfynol.

In some embodiments described herein, suitable dosages of Meparfynol or a pharmaceutically acceptable salt thereof are typically about 0.05 to about 50 mg/day, for example about 0.1 to about 40 mg/day or about 0.2 to about 20 mg/day, preferably about 4 to about 20 mg/day. Optionally, gradually increasing dosages can be administered. That is, treatment can optionally start with relatively low doses which are incrementally increased until a maintenance dose is reached.

In preferred embodiments described herein, Meparfynol or a pharmaceutically acceptable salt thereof may be administered in a single daily dose, or the total daily dosage may be administered as a plurality of doses, (e.g., divided doses two, three or four times daily). Furthermore, Meparfynol or a pharmaceutically acceptable salt thereof may be administered in intranasal form *via* topical use of suitable intranasal vehicles, or *via* transdermal routes, or *via* topical use of ocular formulations, or using those forms of transdermal skin patches well known to persons skilled in the art.

### DOSAGE REGIMEN

Meparfynol, or a pharmaceutically acceptable salt thereof may be administered to an individual in accordance with an effective dosing regimen for a desired period of time or duration, such as at least about one month, at least about 2 months, at least about 3 months, at least about 6 months, or at least about 12 months or longer. In one variation, the compound is administered on a daily or intermittent schedule for the duration of the individual's life.

By way of further example, the pharmaceutical composition comprising Meparfynol, or a pharmaceutically acceptable salt thereof of the present invention may be administered in accordance with a regimen of 1 to 10 times per day, such as once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

In one embodiment described herein, a treatment regimen involving a dosage form of compound, whether immediate release or a sustained release system, may involve administering the compound to the individual in dose of from about 0.1 and about 10 mg/kg of body weight, at least once a day and during the period of time required to achieve the therapeutic effect. In other variations, the daily dose (or other dosage frequency) of Meparfynol as described herein is from about 0.1 to about 8 mg/kg; or from about 0.1 to about 6 mg/kg; or from about 0.1 to about 4 mg/kg; or from about 0.1 to about 2 mg/kg; or from about 0.1 to about 1 mg/kg; or from about 0.5 to about 10 mg/kg; or from about 1 to about 10 mg/kg; or from about 2 to about 10 mg/kg; or from about 4 to about 10 mg/kg; or from about 6 to about 10 mg/kg; or from about 8 to about 10 mg/kg; or from about 0.1 to about 5 mg/kg; or from about 0.1 to about 4 mg/kg; or from about 0.5 to about 5 mg/kg; or from about 1 to about 5 mg/kg; or from about 1 to about 4 mg/kg; or from about 2 to about 4 mg/kg; or from about 1 to about 3 mg/kg; or from about 1.5 to about 3 mg/kg; or from about 2 to about 3 mg/kg; or from about 0.01 to about 10 mg/kg; or from about 0.01 to 4 mg/kg; or from about 0.01 mg/kg to 2 mg/kg; or from about 0.05 to 10 mg/kg; or from about 0.05 to 8 mg/kg; or from about 0.05 to 4 mg/kg; or from about 0.05 to 4 mg/kg; or from about 0.05 to about 3 mg/kg; or from about 10 kg to about 50 kg; or from about 10 to about 100 mg/kg or from about 10 to about 250 mg/kg; or from about 50 to about 100 mg/kg or from about 50 to about 200 mg/kg; or from about 100 to about 200 mg/kg or from about 200 to about 500 mg/kg; or a dosage over about 100 mg/kg; or a dosage over about 500 mg/kg. In some embodiments, a daily dosage of Meparfynol is administered, such as a daily dosage which may include but is not limited to, a daily dosage of about 0.05 mg/kg.

### DOSAGE FREQUENCY

In one embodiment described herein, the dosing frequency can be about a once weekly dosing. In other embodiments described herein, the dosing frequency can be about a once daily dosing. The dosing frequency can be more than about once weekly dosing. The dosing frequency can be less than three times a day dosing. The dosing frequency can be less than about three times a day dosing. The dosing frequency can be about three times a week dosing. The dosing frequency can be about a four times a week dosing. The dosing frequency can be about a two times a week dosing. The dosing frequency can be more than about once weekly dosing but less than about daily dosing. The dosing frequency can be about a once monthly dosing. The dosing frequency can be about a twice weekly dosing. The dosing frequency can be more than about once monthly dosing but less than about once weekly dosing. The dosing frequency can be intermittent (e.g., once daily dosing for 7 days followed by no doses for 7 days, repeated for any 14 day time period, such as about 2 months, about 4 months, about 6 months or more). The dosing frequency can be continuous (e.g., once weekly dosing for continuous weeks). Any of the dosing frequencies can employ any of the compounds described herein together with any of the dosages described herein, for example, the dosing frequency can be a once daily dosage of less than 0.1 mg/kg or less than about 0.05 mg/kg of Meparfynol.

### PSYCHO-PHARMACOLOGICAL TESTS

The usefulness of Meparfynol or a pharmaceutically acceptable salt thereof as described above for use in the treatment of schizophrenia is demonstrated in standard pharmacological test procedures, some of which are outlined as follows. By way of example, compounds of the present invention may be screened *in-vivo* to determine, their capacity, after administration, to affect the behavioural characteristics of the treated subject.

### OPEN FIELD TEST

The Open Field test is designed to measure behavioural responses such as locomotor activity, hyperactivity, and exploratory behaviors. Open Field is also used as a measure of anxiety. Rats and mice tend to avoid brightly illuminated, novel, open spaces, so the open field environment acts as an anxiogenic stimulus and allows for measurement of anxiety-induced locomotor activity and exploratory behaviours. Open field testing is a one trial test with little or no impact on the animal's subsequent behaviour. The apparatus for the Open Field test was a square (100cm by 100cm) made of white Perspex. Each rat is placed in a corner of the field and its behaviour recorded for five minutes. All activity is recorded by a video camera mounted above the open field and scored in real-time (or digitized and scored later) by the advanced motion-recognition software package Ethovision by Noldus, that detects and analyzes the movements of the rat or mouse. The total distance, average speed, rearing/elongation behaviour, and time spent in various parts of the field (such as, for example, the border areas vs. the open, middle area) is measured and analysed. Testing is carried out in a temperature, noise and light controlled room.

### NOVEL OBJECT RECOGNITION (NOR) TEST

This test is useful for evaluating the role of experimental manipulations on cognition. The recognition test is based on the natural tendency of rodents to investigate a novel object instead of a familiar one. The choice to explore the novel object reflects the use of learning and (recognition) memory processes. Novel Object Recognition (NOR) is based on the premise that rodents will explore a novel object more than a familiar one, but only if they remember the familiar one. This tendency is actually shared by humans, as looking time is often used to make inferences about an infant's memory in the absence of explicit, verbal recognition.

### Description I

Before training the animals with objects, they are first allowed to acclimatise to the testing environment, a white Perspex box (30cm by 20cm) equipped with an overhead camera. After an acclimation session, the animals are ready for the training stage. This stage involves the introduction of two identical objects (Lego Duplo) to the environment before allowing the rodent to explore for a period of five minutes: Following the training period, the rodent is removed from the environment for a delay period which can range from 5 minutes to 24 hours, depending on the type of memory being tested. After the delay, the rodent is returned to the arena, where one of the original objects has been replaced by a new one: A glass container.

### Description II

In the two-trial novel object recognition task, a rodent is placed in an enclosure and exposed for a set length of time to two identical objects that are located a specified distance from each other. The rodent is then removed from the environment and a predetermined amount of time is allowed to pass. The subject is then retested in the same environment except that one of the two previously used (familiar) objects is replaced with a novel object that differs from the familiar object in shape, texture and appearance (such as, for example, plastic block is replaced with a metal ball), and the rodent's behaviour is recorded.

The amount of time that the rodents spends exploring each object can be calculated by hand or by using a computer program receiving input from the overhead camera. The literature describes a variety of methods for analyzing results. One technique involves dividing the time spent exploring the novel object by the total time spent exploring either object, yielding % novel exploration. An alternative technique is used to calculate the discrimination ratio, defined as the difference in exploration time for the objects divided by total exploration time. The method of analysis should be suited to the specific experimental setup.

Though simple by design, NOR is actually quite flexible. For instance, changing the duration of the delay period allows one to selectively test short-term or long-term memory. Alternatively, the NOR protocol can be used to selectively test the effects of an acute drug treatment on a specific stage of memory formation. The experimenter can manipulate memory encoding, consolidation or retrieval by injecting the drug prior to the training, delay or testing period, respectively. The desired result is a reduced exploratory time and enhanced spatial learning in response to a test agent in the novel object recognition test.

### PRE-PULSE INHIBITION TEST

The overall goal of this test is to evaluate the effect of an agent on sensory processing (which is a form of working memory).

Prepulse inhibition is a procedure whereby a preceding stimulus attenuates the startle response. The reduced ability to filter out irrelevant auditory stimulation is a characteristic which is thought to contribute to certain manifestations of conditions including inattention, distractibility, and cognitive deficits. The test is quite useful for evaluating transgenic models of schizophrenia as well as to screen potential antipsychotic drugs.

Prepulse Inhibition (PPI) is a neurological phenomenon in which a weaker prestimulus (prepulse) inhibits the reaction of an organism to a subsequent strong startling stumulus (pulse). The stimuli are usually acoustic, but tactile, light, airpuff stimuli are also used. The reduction of the amplitude of startle reflects the ability of the nervous system to temporarily adapt to a strong sensory stimulus when a preceding weaker signal is given to warn the organism. PPI is detected in numerous species ranging from mice to human. Although the extent of the adaptation affects numerous systems, the most comfortable to measure are the muscular reactions, which are normally diminished as a result of the nervous inhibition.

Prepulse inhibition is a cross-species phenomenon (that is, it is present in mammals ranging from mice to humans), yet it is relatively absent among schizophrenic patients and, more recently discovered, among patients with Alzheimer's disease and in people under the influence of drugs, surgical manipulations, mutations. Human studies of PPI have been summarised in a review by Braff et al. in 2001 Human studies of prepulse inhibition of startle: normal subjects, patient groups, and pharmacological studies. Psychopharmacology 2001; 156:234-258). Disruptions of PPI are studied in humans and many other species. The most studied are deficits of PPI in schizophrenia, although this disease is not the only one to cause such deficits. They have been noted in panic disorder (Ludewig, et al., 2005), schizotypal personality disorder (Cadenhead KS, Geyer MA, Braff DL. Impaired startle prepulse inhibition and habituation in patients with schizotypal personality disorder. Am J Psychiatry. 1993 Dec;150(12):1862-7), obsessive-compulsive disorder (Swerdlow et al., 1993), Huntington's disease (Swerdlow NR, Paulsen J, Braff DL, Butters N, Geyer MA, Swenson MR. Impaired prepulse inhibition of acoustic and tactile startle response in patients with Huntington's Disease. J Neurol Neurosur Psychiatry 1995; 58: 192-200*),* nocturnal enuresis and attention deficit disorder (Ornitz *et al.* 1992), and Tourette's syndrome (Swerdlow *et al.* 1994; Castellanos *et al.* 1996). According to one study, people who have temporal lobe epilepsy with psychosis also show decreases in PPI, unlike those who have TLE without psychosis (Morton, N., Gray, N.S., Mellers, J., Toone, B., Lishman, W.A., & Gray, J.A. (1994). Prepulse inhibition in temporal lobe epilepsy. Schizophrenic Research, 15, 191). Therefore, PPI deficits are not typical to specific disease, but rather tell of disruptions in a specific brain circuit.

PPI deficits represent a well-described finding in schizophrenia, with the first report dating back to 1978 (Braff D, Stone C, Callaway E, Geyer M, Glick I, Bali L. Prestimulus effects on human startle reflex in normals and schizophrenics. Psychophysiology. 1978 Jul;15(4):339-43. The abnormalities are also noted in unaffected relatives of the patients. In one study, patients failed to show increased PPI to attended prepulses. Dopamine, which plays a major role in schizophrenia, had been shown to regulate sensorimotor gating in rodent models. These findings fit to the dopamine hypothesis of schizophrenia. In theory, PPI disruption in schizophrenia may be related to the processes of sensory flooding and cognitive fragmentation.

Antipsychotic medications have been shown to increase PPI in patients, with atypical antipsychotics having more effect. Patients display the same gender difference in PPI as healthy people: males have higher PPI compared to females. One notable finding is that patients are specifically deficient in PPI with 60 ms prepulse intervals relative to intervals of other lengths; this remains so even under antipsychotic treatment (see Swerdlow NR, Light GA, Cadenhead KS, Sprock J, Hsieh MH, Braff DL. (2006) Startle gating deficits in a large cohort of patients with schizophrenia. Arch Gen Psych. December, 2006;63:1325-1335).

### Testing for PPI

Murine models are widely used to test hypotheses linking genetic components of various diseases with sensorimotor gating. While some of the hypotheses stand to the test, others are not, as some mice models show unchanged or increased PPI contrary to the expectations, as in the tests of COMT-deficient mice. Certain surgical procedures also disrupt PPI in animals, helping to unravel the underlying circuitry. Many animal studies of PPI are undertaken in order to understand and model the pathology of schizophrenia. Schizophrenia-like PPI disruption techniques in rodents have been classified in one review into four models which are described as follows: (i) PPI impairment driven by dopamine-receptor agonists, most validated for antipsychotic studies; (ii) PPI impairment by 5-HT2 receptor agonists; (iii) PPI impairment by NMDAR antagonists; and (iv) PPI impairment by developmental intervention (isolation rearing, maternal deprivation). Diverse chemical compounds are tested on animals with such deficits. Compounds that are able to restore PPI could be further investigated for their potential antipsychotic role.

An example of one way of testing for PPI: In the prepulse inhibition (PPI) procedure, the rodent is placed in a small chamber and exposed to a brief pulse of noise. The test is used to assess the subject's ability to "gate" or filter environmental information. In the acoustic (startle model) of sensorimotor gating, a weak acoustic stimulus (ie, the prepulse) decreases the reflexive flinching response (startle) produced by a second, more intense, stimulus (the pulse).

The main three parts of the procedure are prepulse, startle stimulus, and startle reflex. Different prepulse-to-pulse intervals, or lead intervals, are used: 30, 60, 120, 240 and 480 ms. Lead interval counts from the start of prepulse to the start of the pulse. With the interval exceeding 500 ms, prepulse facilitation - increased response - is most likely to follow. Burst of white noise is usually used as acoustic startle stimulus. Typical durations are 20 ms for prepulse and 40 ms for pulse. Background noise with 65-70 dB is used in human studies, and 30-40 dB in rodent experiments. Prepulse is typically set 3-12 dB louder than background. Startle response is measured in rodents using the so-called automated "startle chambers" or "stabilimeter chambers", with detectors recording whole-body reaction. In humans, the movements of oculomotor muscles ("eye-blink reflex" or "eye-blink response" assessed using electromyographic recording of orbicularis oculi muscle and by oculography) could be used as a measure. Pulse-alone results are compared to prepulse-plus-pulse, and the percentage of the reduction in the startle reflex represents prepulse inhibition. Possible hearing impairment must be taken into account, as, for example, several strains of mice develop high frequency hearing loss when they mature.

### FORCED SWIM TEST

The Behavioural Despair test (also called the Porsolt test or Forced Swimming test) is a test used to measure the effect of antidepressant drugs on the behaviour of laboratory animals (typically rats or mice). The experimental procedure for the Forced Swim tests differs between rats and mice. Rats are subjected to two trials during which they are forced to swim in an acrylic glass cylinder filled with water, and from which they cannot escape. The first trial lasts 15 minutes. Then, after 24-hours, a second trial is performed that lasts 5 minutes. Mice only require a single experimental session and a single drug administration. The time that the test animal spends without moving in the second trial is measured. This immobility time is decreased by antidepressants. Porsolt RD, Bertin A, Jalfre M. (1977). "Behavioral despair in mice: a primary screening test for antidepressants". Archives Internationales de Pharmacodynamie et de Therapie 229 (2): 327-336. Petit-Demouliere B, Chenu F, Bourin M. (2005). "Forced swimming test in mice: a review of antidepressant activity.". Psychopharmacology (Berl) 177 (3): 245-255.

### Forced Swim Test (FST) in Mice:

### Day 1:

1. Fill a 12 cm diameter glass cylinder to 10 cm with 22°C water.
2. Place two animals simultaneously in individual side-by-side cylinders separated by an opaque screen.
3. Place mice in the cylinders for 6 miutes.
4. Observe the behaviour of the mice for the last 4-minutes of the 6-minute experimental session.
5. Sessions are videotaped and scored blind, Immobility is scored by summing the time spent immobile; movements necessary to maintain the animals head above water were not scored with data from treated groups compared with data from the control group using nonpaired Student's *t* tests (two tailed).
6. Animals are dried with paper towels and heat lamps.

### FST in Rats:

### Habitation session (Day 1)

1. On Day 1, 25 hr prior to testing, place the animals in the experimental room 60 min before the beginning of the habituation session. Randomly assign animals to a drug treatment, but give all animals within a cage the same treatment. Make food and water available throughout the experiment.
2. Weigh two animals individually, then place one rat in each of the two cylinders (20cm in diameter x 40cm high) containing water (25°C) for 15 min (habituation session). No scoring of immobility is performed during the habituation session. This session is needed to habituate the rats to the experimental situation and to induce a stable, high level of immobility during the actual test. Individual weights are used to calculate the dose per animal and for documentation purposes.
3. Remove the rats from the cylinders, dry them with a cloth towel, and place them into a cage.

### Day 2:

1. Repeat day 1 set up.
2. Two animals simultaneously placed in individual side-by-side cylinders separated by an opaque screen and are videotaped. Observe their behavior for 5 min.
3. Score animals for immobility, swimming, and climbing by using a sampling technique to rate the predominant behavior over a 5 second interval (therefore 120 total counts over 10 minutes).
4. Immobility is defined as absence of all movement except motions required to keep the head above the water. Climbing is defined as thrashing movements along the sides of the water tank while swimming behaviour consists of horizontal motion moving from one quadrant of the water tank to another.

### WATER MAZE SPATIAL LEARNING TEST

The Morris water maze (MWM) is a test of spatial learning for rodents that relies on distal cues to navigate from start locations around the perimeter of an open swimming arena to locate a submerged escape platform. Spatial learning is assessed across repeated trials and reference memory is determined by preference for the platform area when the platform is absent.

The test apparatus consists of a large circular pool (1m diameter, 80cm high, temperature 26°C) with a platform (11cm diameter) submerged 1.5cm below the surface. Both the pool and the platform were constructed of black polyvinyl plastic, which offered no intra-maze cues. The experimental room contained several extra-maze visual cues. During training the platform was hidden in the same quadrant 30cm away from the edge of the maze. There were five trials, each of them beginning with the rat facing the wall of the maze in one of three different locations. The time taken for the rat to find the platform within a 90s period was recorded. Probe trials where the platform was removed from the maze and the time the animal spent in the target quadrant were also performed.

Reversal and shift trials enhance the detection of spatial impairments. Trial-dependent, latent and discrimination learning can be assessed using modifications of the basic protocol. Search-to-platform area determines the degree of reliance on spatial versus non-spatial strategies. Cued trials determine whether performance factors that are unrelated to place learning are present. Escape from water is relatively immune from activity or body mass differences, making it ideal for many experimental models. The MWM has proven to be a robust and reliable test that is strongly correlated with hippocampal synaptic plasticity and NMDA receptor function.

As the Examples demonstrate, Meparfynol (also known as UCD-1216) significantly enhanced the pre-pulse inhibition response, reduced exploratory time in the novel object recognition paradigm and enhanced spatial learning. Thus, Meparfynol combines a cognitive enhancing action with an improved prepulse inhibitory action which is indicative of an enhanced sensory processing action. These results indicate that Meparfynol may be useful in the effective treatment of symptoms associated with psychosis, schizophrenia, a cognitive impairment disorder, a mood disorder, a nervous system disorder and/or a behavioural disorder in a subject in need of such treatment.

### PSYCHO-PHARMACOLOGICAL TESTS TO MEASURE BEHAVIOURAL RESPONSES IN HUMANS

### Human Equivalents of the Novel Object Recognition Test

Novel object recognition (NOR) is a memory test based on the differential exploration of familiar and new objects. According to the authors who originally devised the test (Ennaceur and Delacour, 1988 A new one-trial test for neurobiological studies of memory in rats. 1, Behavioral data. Behavioural Brain Research 31, 47-59), it has several interesting characteristics: (a) It is similar to visual recognition tests widely used in subhuman primates, this allows interspecies comparisons; (b) It is entirely based on the spontaneous behaviour of rats and can be considered as a 'pure' working-memory test completely free of reference memory component; and (c) It does not involve primary reinforcement such as food or electric shocks; this makes it comparable to memory tests currently used in man. For our interpretation of NOR test, we focused primarily on its ability to evaluate the effect a drug has on working memory. There are several tests used to evaluate working memory in humans, some of the main one are outlined below:

### Visuo-spatial delayed response tasks

This task involves presentation of a visual cue (a black dot) on a computer screen. The cue is removed for a delay of either 0 or 8 seconds. After the delay period, the subjects indicate the screen location of the cue with a fine-pointed light pen. Improvements in working memory can be assessed by checking the accuracy of pin-pointing the location of cue after 8 seconds.

### Card Sorting Tasks

These tasks assess a person's ability to 'set shift' i.e. the ability to display flexibility in the face of changing schedules of reinforcement. A well-known version of this task is known as the Wisconsin Card Sorting Test or WCST. A WCST deck is made up of 128 response cards, and 4 stimulus cards. Each stimulus card has a different number, colour, and shape of symbol: a red triangle, two green stars, three yellow crosses, and four blue circles. The response cards each have a different combination of those parameters, one has four red crosses, another has two yellow circles, and so forth. At the beginning of the test, the experimenter places the four stimulus cards on the table, and tells the subject that he is to sort the cards in the response deck on to each pile. This is purposefully ambiguous in order to make sure the subject will make incorrect placements, making it possible to tell how well the subject is picking up sorting rules during the game. The subject is also warned that the rules of sorting will change during the experiment. From then on the experimenter answers only "right" or "wrong" to each card placement by the subject. Since there are only three possible characteristics to judge by (number, colour, and shape), the experimenter can only change sorting rules twice. With each change of sorting rule, the experimenter watches to see how long it takes the subject to figure out the rules have changed, how long it takes him to learn the new rules, and what mistakes he makes while learning them.

### Associative memory tasks

There are several types of associative memory test performed in psychological testing, a sample test would be the fan effect paradigm. Is this test subjects have to memorize a series of sentences which encode conflicting information about the associations among various elements (e.g. 'The doctor is in the house' and 'The doctor is in the park'). This task measures susceptibility to interference in memory, as well as the ability to maintain associations under conditions of interference.

### Context memory tasks

The ability to associate items in memory has been linked to prefrontal function in a variety of context memory studies. A typical context memory task is where subjects are shown two lists of words, and later have to remember on which of the two lists each word has originally appeared

### The Stroop Task

In psychology, the Stroop effect is a demonstration of interference in the reaction time of a task. When a word such as blue, green, red, etc. is printed in a colour differing from the colour expressed by the word's semantic meaning (e.g. the word "red" printed in blue ink), a delay occurs in the processing of the word's colour, leading to slower test reaction times and an increase in mistakes. This can be used to assess working memory as it is or by modifying the test by adding conditions. A typical test could include two conditions: such as naming the color of a series of color blocks, and naming the color of a series of conflicting colour words (e.g. 'red' printed in blue ink). The difference in time to complete the two tasks was can be used as a measure of interference.

### Cognitive Function Tests in Humans

Where a neurodegenerative disorder affects a cognitive ability, a subject can be diagnosed by any one of a number of standardized cognitive assays, e.g., the Mini-Mental State Examination, the Blessed Information Memory Concentration assay, or the Functional Activity Questionnaire. See, e.g., Adelman et al. (2005), Am. Family Physician, 71(9):1745-1750. Indeed, in some cases a subject can also be diagnosed as having a high risk of developing a chronic neurodegenerative condition (e.g., Alzheimer's disease), even in the absence of overt symptoms. For example, the risk of Alzheimer's disease in a subject can be determined by detecting a decrease in the volumes of the subject's hippocampus and amygdala, using magnetic resonance imaging. See, e.g., den Heijer et al. (2006), Arch Gen Psychiatry, 63(1):57-62. Assay of prognostic biomarkers in a sample from a subject are also useful in prognosis or diagnosis of a chronic neurodegenerative condition. For example, where the chronic neurodegenerative condition is Alzheimer's disease, prognostic biomarkers include, but are not limited to, total tau protein, phosphotau protein, β-amyloidₜ.4₂ peptide, β-amyloidi-₄₀ peptide, complement component 1, q subcomponent (CIq) protein, interleukin 6 (IL-6) protein, apolipoprotein E (APOE) protein, o-1-antichymo trypsin protein, oxysterol (e.g., 24S-hydroxycholesterol), isoprostane (e.g., an F2-isoprostane), 3-nitrotyrosine, homocysteine, or cholesterol, or any combination thereof, e.g., the ratio of β-amyloidi.42 peptide to β-amyloidτ.40 peptide.

### PSYCHO-PHARMACOLOGICAL FUNCTIONAL ACTIVITY

The functional pharmacological activity of Meparfynol or a pharmaceutically acceptable salt thereof at a given receptor can be achieved by a variety of *in-vitro* methodologies. A currently favoured assay is the Receptor Selection and Amplification Technology (R-SAT) assay disclosed in US 5,707,798, the content of which is hereby incorporated by reference in its entirety. Another currently favored assay is the PI Hydrolysis assay (see, for example, the description of the PI Hydrolysis assay in Barker et al (1994) J Biol Chem 269 (16); 11687-11690. Defining the ability of Meparfynol or a pharmaceutically acceptable salt thereof to penetrate the blood brain barrier and elicit a meaningful biological response can be achieved by a variety of methodologies. A currently favored assay is the hippocampal MAP kinase activation assay.

The suitability of Meparfynol, or a pharmaceutically acceptable salt thereof can be readily determined by evaluation of its potency and selectivity using literature methods followed by evaluation of various pharmacological studies which include but are not limited toxicity, absorption, distribution, metabolism, excretion (ADME) and pharmacokinetic studies which are carried out in accordance with standard pharmaceutical practice.

Selectivity ratios may readily be determined by the skilled person in the art. For some applications, Meparfynol, or a pharmaceutically acceptable salt thereof or mixtures thereof has at least about a 25, 50, 75, 100 fold selectivity to the desired target, preferably at least about a 150 fold selectivity to the desired target, preferably at least about a 200 fold selectivity to the desired target, preferably at least about a 250 fold selectivity to the desired target, preferably at least about a 300 fold selectivity to the desired target, preferably at least about a 350 fold selectivity to the desired target.

Receptor affinity is a good starting point for determining the mechanism of a particular compound. Receptor affinity may readily be determined by the skilled person in the art. For most applications, Meparfynol, or a pharmaceutically acceptable salt thereof or mixtures thereof has an affinity for its target of at least 10⁻⁷M, preferably 10⁻⁸M, preferably 10⁻⁹M.

In this regard, IC50 values and EC50 values for Meparfynol, or a pharmaceutically acceptable salt thereof or a mixture thereof may be determined using established literature methodology.

As used herein, the term "IC50" means the concentration of drug that is required to inhibit the action, binding, or activity of some other drug by 50%.

As used herein, the term "EC50" is the dosage at which the desired response is present for 50 percent of the population. It is commonly used as a measure of drug potency.

For some applications, Meparfynol, or a pharmaceutically acceptable salt thereof has an EC50 value of less than 300nM, 250nM, 200nM, 150nM, preferably less than about 100 nM, preferably less than about 75 nM, preferably less than about 50 nM, preferably less than about 25 nM, preferably less than about 20 nM, preferably less than about 15 nM, preferably less than about 10 nM, preferably less than about 5 nM.

### ANIMAL MODELS

*In vivo* models may be used to investigate and/or design therapies and/or therapeutic agents to treat symptoms associated with schizophrenia. The models could be used to investigate the effect of various tools/lead compounds on a variety of parameters which indicate the psycho-pharmacological behavioural response as defined, for example, in any one or more of the above described psycho-pharmacological tests.

The disclosure further provides transgenic nonhuman animals capable of expressing a nucleotide sequence encoding, for example, a specific gene associated with schizophrenia or a variant, homologue, derivative or fragment thereof and/or a transgenic nonhuman animal having one or more nucleotide sequence encoding, for example, a specific gene associated with schizophrenia invention or a variant, homologue, derivative or fragment thereof inactivated.

Expression of such a nucleotide sequence is usually achieved by operably linking the nucleotide sequence to a promoter and optionally an enhancer, and microinjecting the construct into a zygote. See Hogan et al., "Manipulating the Mouse Embryo, A Laboratory Manual,"Cold Spring Harbor Laboratory. Inactivation of such a nucleotide sequence may be achieved by forming a transgene in which a cloned nucleotide sequence is inactivated by insertion of a positive selection marker. See, for example, Capecchi, Science 244,1288-1292 (1989). The transgene is then introduced into an embryonic stem cell, where it undergoes homologous recombination with an endogenous variant gene. Mice and other rodents are preferred animals. Such animals provide screens and/or screening systems for identifying compounds capable of modulating the psycho-pharmacological behavioural response, as defined, for example, in any one or more of the above described psycho-pharmacological tests.

One exemplary model for testing the activity of Meparfynol, or a pharmaceutically acceptable salt thereof or a mixture thereof as described herein to treat and/or prevent and/or delay the onset and/or development of schizophrenia employs phencyclidene, which is chronically administered to the animal (e.g., non-primate (rat) or primate (monkey)), resulting in dysfunctions similar to those seen in schizophrenic humans. See Jentsch et al., 1997, Science 277:953-955 and Piercey et al 1988, Life Sci. 43(4):375-385). Standard experimental protocols may be employed in this or in other animal models.

### TESTING IN HUMANS

Preferred combinations of (1) Meparfynol, or a pharmaceutically acceptable salt thereof and (2) (i) an NMDA/AMPA receptor modulator; (ii) a nootropic agent; (iii) an anti-depressive agent (eg SSRI); (iv) an Agonist/Antagonist/Partial Agonist/Partial Antagonist; (v) an anti-epileptic agent; (vi) a neuroleptic/neuropsychiatric agent; (vii) an atypical anti-psychotic agent; (viii) a cholinergic enhancer; and (ix) an attention stimulant/non-stimulant useful herein can also be tested clinically, typically orally, in humans as well as in an animal model. Each component is administered singly at different times to a population of human subjects, each component being administered in an amount which produces little or no response, typically less than a 50% response. By administering each component singly, it is meant that one component is administered, followed at a later time by the second component after having allowed an appropriate time for washout of the first component. After the washout period for each component administered singly, the components are co-administered in a manner such that both components co-operate pharmacokinetically, preferably such that the peak pharmacokinetic effect due to each coincides. Co-administration is evaluated according to the parameters mentioned above and by questionnaires, thereby providing a basis for comparison of the effects of co-administration with that for each single administration.

### BRIEF DESCRIPTION OF THE FIGURES

The invention is now further described only by way of example in which reference is made to the following Figures:
Figure 1 provides a schematic diagram of the administration regime for the compound of the invention under investigation;
Figure 2 provides a pictorial image of the set-up for the Forced Swim Test;

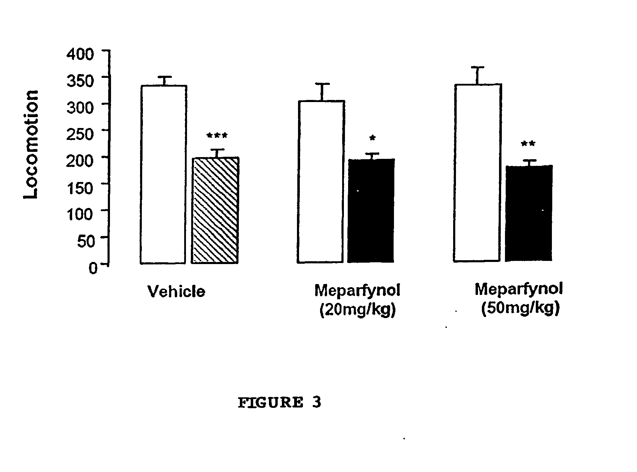

Figure 3 is a graph which shows the influence of Meparfynol (20 mg/kg and 50mg/kg) on mouse locomotor activity over two days in an open field arena;

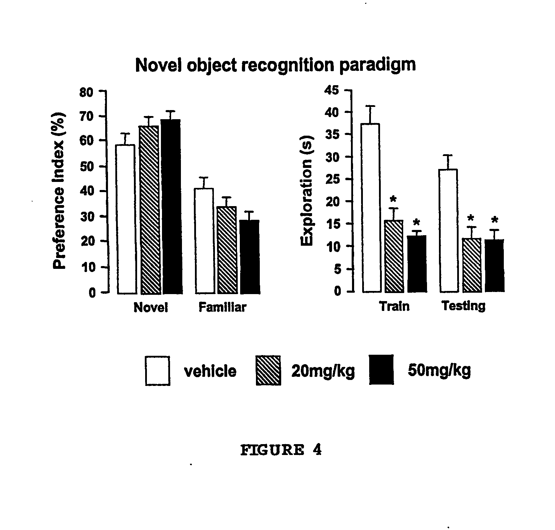

Figure 4 provides graphs which show the influence of Meparfynol during the Novel Object Recognition (NOR) test in mice. During the training phase (day 1), animals are presented with two identical objects, and the % exploratory time spent at each object computed as a preference index (PI). During the recall or testing phase, on day 2, a novel object and familiar object are presented with a bias for the novel object expected (left hand panel). Meparfynol treated animals also spend less time exploring objects in both the training (day 1) and testing phases (day 2) (right hand panel).

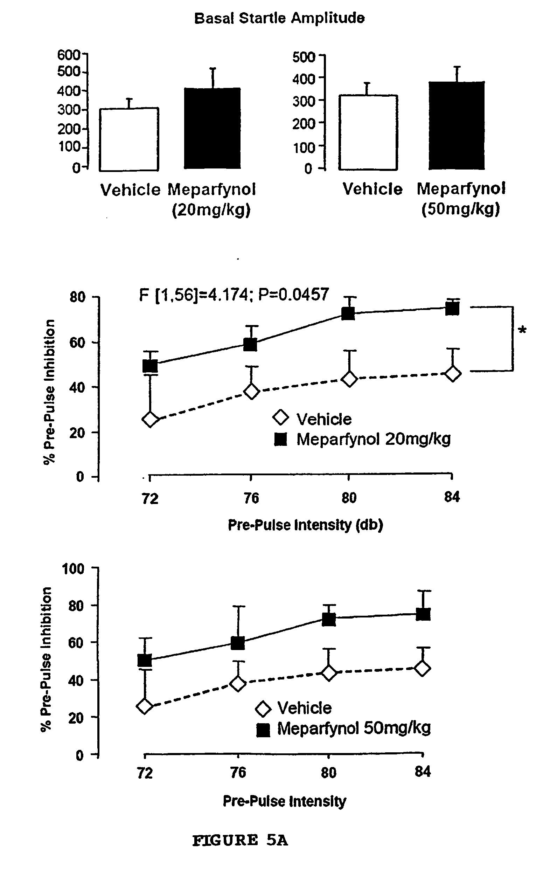

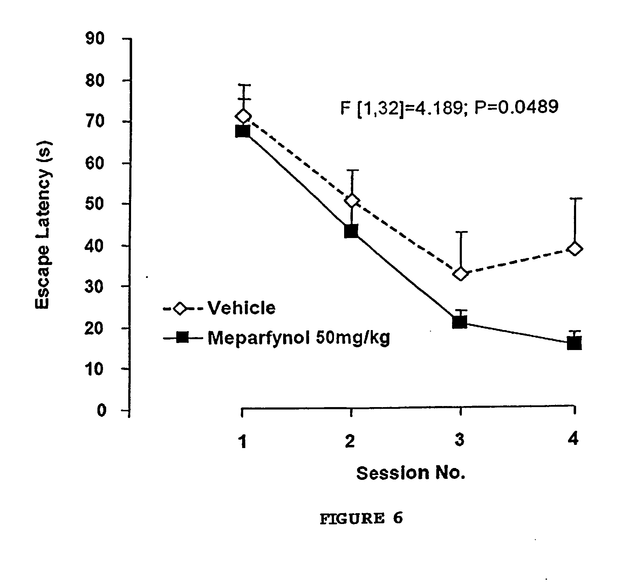

Figure 5A shows the influence of Meparfynol in mice on pre-pulse inhibition (PPI) of the startle response and Figure 6b shows similar data generated in rats reared in isolation. The startle response is the contraction of the whole body musculature in response to a sudden, loud noise. The magnitude of the response can be reduced when preceded by a weak noise (pre-pulse). This reduction or inhibition of startle is not observed in schizophrenic patients. Meparfynol at 20mg/kg significantly enhances the pre-pulse inhibition of startle response;

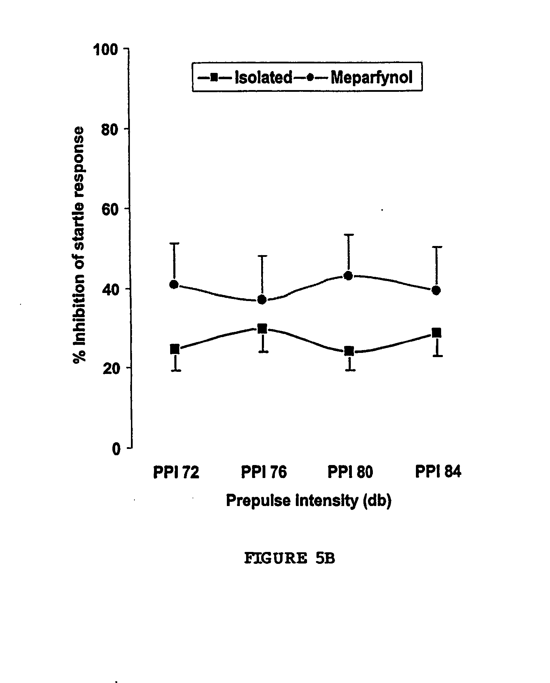

Figure 5B shows the influence of Meparfynol on the pre-pulse inhibition (PPI) of the startle response in isolated animals (rats). This reduction or inhibition of startle is not observed in the isolated animals. Meparfynol at 20 mg/kg significantly enhances the pre-pulse inhibition of startle response (p < 0.05, two-way ANOVA).
Figure 6 is a graph which shows the performance of Meparfynol treated animals (mice) in the water maze. The water maze is used as a test of spatial memory. In it, animals learn the location of a hidden platform in a circular pool of water. Multiple sessions of training in the water maze are directed at detecting drug effects on behaviour and cognition across many trials of the paradigm;

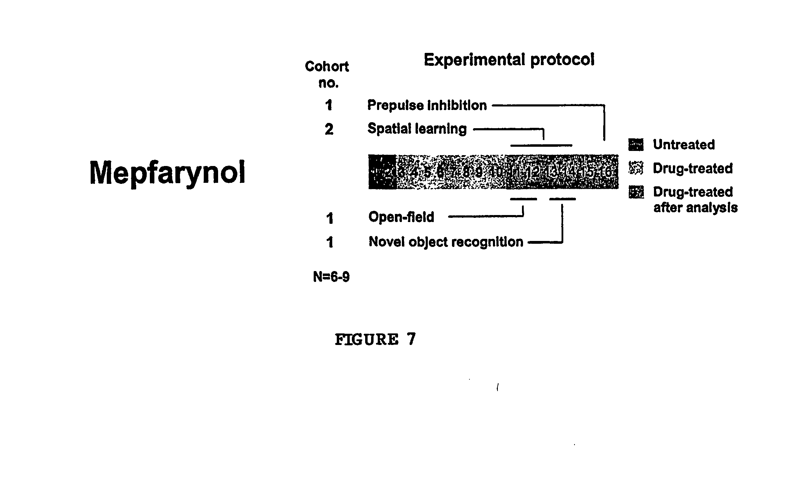

Figure 7 provides a schematic diagram which shows the experimental protocol employed in the behavioural evaluation of Meparfynol;
Figure 8A displays the two dimensional chemical structure of Meparfynol (Formula II);
Figure 8B displays the pharmacokinetic data for Meparfynol in mice. This panel displays the blood concentration of the drug (in ng/ml) at increasing time points (the time points were 20mins apart) following a single injection of the compound at a dose of 50mg/kg into the intraperitoneal cavity. The drug concentration in the blood samples was determined using GC mass spectroscopy; and
Figure 8C displays the top ranking receptors for which Meparfynol displayed affinity. Affinities were determined by having a 1µM concentration of Meparfynol compete for receptor binding with a reference ligand (a compound with a known affinity for the receptor in question). The amount of reference ligand that is prevented from binding to a particular receptor is measured and this gives rise to the "percent target inhibition" value that is displayed in this panel.

### EXAMPLES

The following Examples are provided to illustrate but not limit the invention.

### MEPARFYNOL

The drug, with a IUPAC classification of 3-methylpent-1-yn-3-ol, is known under the non-proprietary name, Meparfynol, was taken as representative of the compounds described herein.

The chemical formula for Meparfynol is C₆H₁₀O and its CAS number is 77-75-8. The compound is also known as 3-Methyl-1-pentyn-3-ol, 3-Methyl-1-pentyn-3-ol, and 3-Methyl-1-pentin-3-ol and under the following synonyms 1-Pentyn-3-ol, 3-methyl-, Allotropal, Apridol, Atempol, ¦Á-Ethyl-¦Á-methylpropargyl alcohol, Citodorm, Dormiphen, Dormidin, 3-Methyl-1-pentin-3-ol, Dormigen, Dormison, Dormosan, Formison, Hexofen, Imnudorm, Melpintol, Methylparafynol, Methylpentanol, Oblivon, Pentadorm, Pentinol, Pentydorm, Perlopal, Riposon, Sedapercut, Seral, Somnesin, 2-Butanol, 2-ethynyl-, 3-Methyl-1-pentyn-3-ol,, anti-Stress, m-Pentynol, Aniphor, Atemorin, BDH, Comesa, Dalgol, Dorison, Dormalest, Dormocit, Ethinylmethylethylcarbinol, Hesofen, Insomnol, Macarol, Mecarol, Mepentamato, Methylethylacetylenylcarbinol, Mepentil, Methylethylethynylcarbinol, Methylpentynolum, Metilparafinolo, Metilpentinolo, Miramel, N-Oblivon, Noxokratin, Oblevil, Oblivon C, Olosot, Olvadon, Pentyrest, Placidal, Sintyal, Sonnormon, Trusono, UTIL, 2-Ethinyl butanol-2, 2-Ethinylbutanol, 3-Ethylbutanol, 3-Methyl-1-pentynol, 3-Methylpentyn-3-ol, Methylpentinol, 2-Aethinylbutanol, 3-Ethylbutinol, 3-Ethylbutynol, Ethyl ethynyl methyl carbinol, 3-Methyl-pentin-(1)-ol-(3), 3-Methylpentin-3-ol, Methylpentynol, 3-Metil-pentin-3-ol, Pent-1-yne-3-ol, 3-methyl-, 2-Ethynyl-2-butanol, Anti-stress, 3-Methylpent-1-yn-3-ol

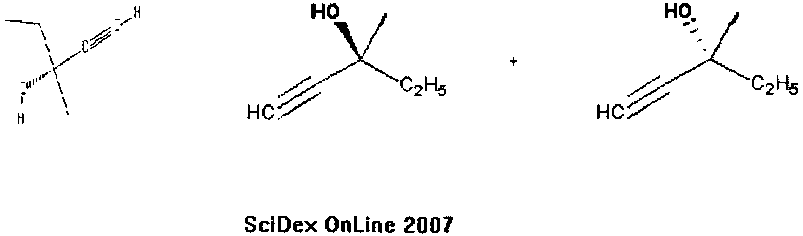

### MEPARFYNOL

Experimental protocol employed in the behavioural evaluation of Meparfynol.

### EXAMPLE 1 - MATERIALS AND METHODS I

The Experimental protocol employed in the behavioural evaluation of Meparfynol is outlined in Figure 7. Two separate cohorts of C57B16 mice were employed. Cohort 1 was used to evaluate the drug effect on prepulse inhibition, open-field and novel object recognition. Cohort 2 was used to evaluate drug effects on spatial learning. The behavioural tests were administered in sequence as per day number indicated in the bar. The drug was administered by the intraperitoneal route and on the day of training the drug was administered after the behavioural analysis. Meparfynol/UCD-1216 was administered at doses of 20 and 50mg/kg. The compounds were administered once daily, via the intraperitoneal route, for 7 days prior to testing and the animals were drug-free at time of training.

### PSYCHO-PHARMACOLOGICAL TESTS

### Neurobehavioural screening methods

A modified SHIRPA protocol was adopted in order to provide an *in vivo* drug profile. The SHIRPA protocol is used in both academic and industrial environments for an initial behavioural characterisation of potential psychopharmaceuticals. The first behavioural tier analysis of our modified screen comprised open-field (Figure 3), novel object recognition (NOR; Figure 4), and pre-pulse inhibition (PPI; Figures 5A and 5B) paradigms. These psycho-pharmacological tests are described in more detail below:

### FIRST BEHAVIOURAL TIER ANALYSIS

### Open Field Analysis - potential anxiolytic behaviour

Open-field analysis was used to indicate drug effects on locomotion, grooming and rearing activities. Furthermore, by determining how much time is spent in the centre of the open-field potential anxiolytic-like behaviour could be identified.

### Novel Object Recognition (NOR)

Novel object recognition was used to assess drug influence on visual recognition memory and attention.

### Pre-Pulse Inhibition - sensory processing (working memory)

Pre-pulse inhibition was used to evaluate drug effects on sensory processing, a form of working memory.

### SECOND BEHAVIOURAL TIER ANALYSIS

### Forced Swim Test (FST) - potential antidepressant

This determines potential antidepressant (forced swim test; FST) actions. The forced swim tests the time spent immobile as a measure of despair.

### THIRD BEHAVIOURAL TIER ANALYSIS PHASE

### Water maze spatial learning

Finally, drugs inducing neurobehavioural profile are evaluated in the water maze spatial learning paradigm, a robust task of information acquisition and consolidation - a measure of cognitive action

### MATERIALS AND METHODS II

The influence of Meparfynol on pre-pulse inhibition in mice is shown in Figure 5A. Mepfarynol was administered by the intraperitoneal route at the doses indicated and as per details outlined above. Values were analysed using a two way ANOVA and Student t-test and those with a p value <0.05 were accepted as significant and where appropriate are indicated with an asterisk.

One of the most robust, non-invasive rodent models used for studies relating to schizophrenia is isolation rearing of rats (Weiss and Feldon, 2001; Fone and Porkess, 2008). This model exhibits a deficit in pre-pulse inhibition (PPI), the normal reduction in startle response following a low intensity pre-stimulus. PPI is used as an operational measure of sensorimotor gating and is known to be defective in schizophrenics (Swerdlow et al., 1994; Ludewig et al., 2002). Figure 5B shows the results of a study on rats reared in isolation measuring the influence of Meparfynol on the pre-pulse inhibition (PPI) of the startle response. Reduction or inhibition of startle is not observed in the isolated animals control. Administration of Meparfynol at 20 mg/kg significantly enhances the pre-pulse inhibition of startle response (p < 0.05, two-way ANOVA).

Weiss IC, Feldon J (2001) Environmental animal models for sensorimotor gating deficiencies in schizophrenia: a review. Psychopharmacology 156, 305-326.

Fone KCF, Porkess MY (2008) Behavioural and neurochemical effects of post-weaning social isolation in rodents-Relevance to developmental neuropsychiatric disorders. Neurosci. Biobehav. Rev., doi:10.1016/j.neubiorev.2008.03.003

Swerdlow NR, Braff DL, Taais N, Geyer MA (1994) Assessing the validity of an animal model of deficient sensorimotor gating in schizophrenic patients. Arch. Gen. Psychiat. 51, 139-153.

Ludewig K, Geyer M, Etzensberger M, Vollenweider FX (2002) Stability of the acoustic startle reflex, prepulse inhibition, and habituation in schizophrenia. Schizophr. Res. 55, 129-137.

### MATERIALS AND METHODS III

The structure (Figure 8A), pharmacokinetics (Figure 8B) and receptor affinity (Figure 8C) of Mepfarynol are shown in Figure 8. Analysis of receptor affinities was performed by Novascreen™ (www.novascreen.com) and those displacing >20% of the natural ligand are indicated by the filled boxes. Blood levels of Mepfarynol were determined by GC mass spectroscopy in separate samples taken from a cannulated jugular at increasing time intervals following a single intraperitoneal injection of the drug (50mglkg).

### INTRAVENOUS ADMINISTRATION/MULTIPLE TIME POINT PROCEDURE FOR RATS

This protocol employs a traditional pharmacokinetic approach. Groups of animals are dosed with the compound of interest, blood samples are obtained, and animals are sacrificed at predetermined times for brain tissue analysis.

NOTE: This procedure involves rodent survival surgery. Sterile surgical procedures must be employed, as specified by prevailing animal care regulations. All surgical instruments and consumable items used for surgery must be sterile.

### PHARMACOKINETICS

### A. Prepare rat for surgery

1. Anesthetize a 200- to 350g laboratory rat with an appropriate anesthetic to maintain proper anesthesia for a surgical procedure of ≤1 hr.
2. Administer appropriate antibiotic to the animal.
3. Place anesthetized rat right side up (lying on its belly) on a warming pad. Shave the fur from its back, between the shoulder blades, using an animal fur shaver. Clean the surgical area with an iodine solution. Using a pair of 4.5-in. operating scissors, make a small incision (0.3 cm long) between or slightly ahead of the shoulder blades.
4. Reposition the rat so that it is upside down (lying on its back) on the warming padwith its head located towards the surgeon and its tail away from the surgeon.
5. Shave the fur from its neck and shoulder areas. Wash surgical area with an iodine solution and cover area with sterile drapes, leaving only the surgical site exposed.

### B. Perform surgery

6. Using a scalpel, make a 2- to 3-cm-long incision over the skin site where pulsation of the right jugular vein can be observed.
7. Using a pair of 4-in. full or strongly curved microdissecting forceps, bluntly separate the subcutaneous and muscle tissues to expose a section of the jugular vein.
8. Using the forceps, carefully clean the vessel of connective tissue from the point of the chest muscle penetration (towards the heart) to a point where it bifurcates into two smaller vessels.
9. Using a sterile 4.0 suture without needle, ligate the vein shut at the point of the bifurcation (towards the snout).
10. Subcutaneously tunnel a 6-in. eye probe from the incision in the back (between the shoulder blades) to the incision in the neck and shoulder area. Insert a jugular vein catheter into the eye of the probe. Pull the probe out so that the catheter is subcutaneously tunneled under the skin, such that the catheter end extends out from the incision on the back and the silastic tubing end extends from the jugular vein incision.
11. Position the catheter so that it lies comfortably in the jugular vein incision site. Cut the silastic tubing such that the free end is about 0.5 cm short of the midline of the front paws and the silastic-PE50 junction rests over the jugular vein. Make a beveled cut at the end of the silastic tubing.
12. Connect the PE50 tubing of the catheter to a 22-G hypodermic needle with the beveled end cut off, affixed to a 3-ml syringe containing prewarmed sterile saline. Fill the catheter with sterile saline.
13. Place two or three more pieces of 4.0 suture under the jugular vein, towards the heart. These pieces will be used to secure the catheter in the vein once the catheter is properly positioned.
14. Using a pair of 3-in. Vannas spring scissors, make a small, nicking incision in the jugular vein. Be careful not to cut through the vein.
15. Insert one point of a pair of Dumont no. 7 microdissecting forceps into the jugular vein incision. Slide the beveled end of the catheter along the probe into the incision. Continue inserting and guiding the catheter into the vein until the cut end of the vein covers the juncture of the silastic and PE50 tubing.
16. To test for catheter patency, inject 0.3 ml sterile saline and then slowly pull back on the syringe plunger to withdraw blood.
17. Clear the catheter of blood by flushing with 0.5 ml sterile saline.
18. Anchor the catheter to the vein by tying the 4.0 sutures (step 13) securely (but not too tightly) around the vein and the PE50 tubing.
19. Suture the catheter to the muscle bed using a 5.5-in. Olsen-Hegar needle holder and sterile 4.0 suture with needle.
20. Close the internal incision by suturing the muscle and subcutaneous tissues with 4.0 sutures.
21. Close the skin incision using a sterile 2.0 suture with needle.
22. Place anesthetized animal right side up (on its belly). Suture the exteriorized catheter to the skin and then close the incision on the back using 2.0 suture.
23. Trim the exteriorized PE50 tubing so that no more than 2.5 to 3 cm is exposed. Plug the open end of the catheter with a sterile piece of 22-G stainless steel wire.
24. Apply topical antibiotics to all incision sites and allow animal to recover from the anesthetic using approved postoperative care protocols.
25. Slowly flush the jugular vein catheter with 0.5 to 1.0 ml sterile 10 U/ml heparinized saline each day to maintain patency.

At least 7 Days are allowed to elapse between the surgery and experimental study.

### C. Prepare experimental equipment

26. Label an appropriate number of 1.5-ml microcentrifuge tubes with caps for the number of desired blood samples per rat.
27. Fill a 1-, 3-, or 5-ml syringe (depending on the dosing volume) with prewarmed test compound solution for each animal to be studied. To each syringe attach a 22-G hypodermic needle with the beveled end cut off. Keep the syringe and its contents warm by wrapping it in a preheated warming pad.
28. Prepare an appropriate number of 1-ml syringes filled with 1 ml prewarmed sterile saline.
29. Prepare an appropriate number of empty 1-ml syringes for blood collection.

### D. Perform experimental study

30. Transfer the catheterized rat to an appropriate animal holding cage.
31. Connect one end of a 20-cm-long piece of PE50 tubing to a 1-ml saline-filled syringe using a 22-G hypodermic needle with the beveled end cut off. Fill tubing with sterile saline and connect the free end of the saline-filled PE50 tubing to the exteriorized jugular vein catheter using a 22-G stainless steel connector. Gently flush catheter with 0.3 ml saline.
32. Withdraw a predose blood sample (blank; time zero) by gently pulling back the syringe plunger so that the luer-lock needle hub is filled with blood. Attach a fresh syringe (step 30) to the luer-lock hub and collect a 0.1- to 0.15-ml blood sample.
35. Remove the sample syringe, transfer sample to the prepared vial (step 27), and reattach the first syringe (filled with saline and possibly mixed with blood) to the needle hub. Flush the PE50 line and refill it with 0.3 to 0.5 ml sterile saline. Replace the initial syringe with a fresh syringe containing sterile saline.
36. Attach the test compound dosing syringe (step 28) to the PE50 tubing. Administer the dose intravenously over 30 to 60 sec. Remove the dosing syringe and attach a saline-filled syringe to the PE50 line. Flush the residual dose intravenously with saline and refill the tubing with sterile saline. Retain a 0.25-ml aliquot of the test compound solution for later analysis.
37. Withdraw blood samples at predetermined time intervals.
38. Withdraw the last blood sample from about 30 to 60 sec before sacrifice, quickly anesthetize the rat, and decapitate it.

### DISCUSSION OF RESULTS I and II

Meparfynol showed little effect in the open-field paradigm (Figure 3) and only exhibited a modest enhancement of working memory as measured by the NOR test, as judged by the increased exploratory bias for the novel object (Figure 4). However, Mepfarynol significantly reduced novel object exploratory time, potentially indicating increased awareness and faster information processing, as compared to the vehicle-treated controls. This enhanced sensory processing effect was also confirmed in the prepulse inhibition and water maze paradigms (Figures 5A, 5B and 6). The significant sensory processing actions of Meparfynol, observed in both the novel object recognition and pre-pulse inhibition paradigms, suggests this compound may provide benefit in conditions of impaired sensory processing, as in schizophrenia, and that this would summate with its significant pro-cognitive actions, as observed in the water maze paradigm.

### RESULTS III - Pharmacokinetics for Meparfynol

Figure 8C displays the top ranking receptors for which Meparfynol displayed affinity. Affinities were determined by having a 1µM concentration of Meparfynol compete for receptor binding with a reference ligand (a compound with a known affinity for the receptor in question). The amount of reference ligand that is prevented from binding to a particular receptor is measured and this gives rise to the "percent target inhibition" value that is displayed in this panel. The higher the target inhibition value, the greater the affinity Meparfynol has for this particular receptor as it displaces a greater proportion of the reference ligand. This assay is an automated high throughput assay performed on cell lines that express only one type of receptor and was carried out by Novascreen (www.novascreen.com).

As Figures 8C demonstrates, the target inhibition values for Meparfynol are quite low (all less than 20%), so it is not possible to draw conclusions as to the mode of action of Meparfynol from these results.

### OVERALL SUMMARY

Meparfynol (Figures 3-6) exhibited a wide range of effects in a wide range of the neurobehavioural paradigms evaluated. In particular, UCD-1216 (Meparfynol) significantly enhanced the pre-pulse inhibition response, reduced exploratory time in the novel object recognition paradigm, enhanced spatial learning and reduced neophobia (fear of anything new). These findings conclude UCD-1216 is capable of enhancing sensory processing actions. It is of interest that these findings reflect a "chronic" administration protocol (1 dose/day x 8 days prior to testing) as opposed to an "acute" administration protocol where only one dose would be given prior to testing).

## Claims

1. 3-methylpent-1-yn-3-ol (meparfynol) or a pharmaceutically acceptable salt thereof for use in the treatment of schizophrenia in a subject in need of such treatment.

2. A pharmaceutical composition comprising a pharmaceutically acceptable carrier and, as an active ingredient, an effective amount of a compound according to Claim 1 for use in the treatment of schizophrenia.

3. Use of 3-methylpent-1-yn-ol (meparfynol) or a pharmaceutically acceptable salt thereof in the manufacture of a medicament for use in the treatment of schizophrenia in a subject in need of such treatment.

4. Use of the compound of Claim 1 or a pharmaceutically acceptable salt thereof for manufacturing a medicament for slowing the progression of schizophrenia in an individual who has been diagnosed with schizophrenia; or preventing or delaying development of schizophrenia in an individual who is at risk of developing schizophrenia.

5. Use of the compound of Claim 1 or a pharmaceutically acceptable salt thereof for manufacturing a medicament for alleviating schizophrenia in a subject in need of such treatment.

6. Use of the compound of Claim 1 or a pharmaceutically acceptable salt thereof for manufacturing a medicament for preventing and/or treating and/or delaying the onset and/or development of schizophrenia and/or decreasing the intensity or severity of schizophrenia in an individual and/or enhancing the quality of life of an individual (i) who is diagnosed with schizophrenia; (ii) who is considered at risk for developing schizophrenia (e.g., an individual whose one or more family members have had schizophrenia); (iii) who has been diagnosed as having a genetic mutation associated with schizophrenia or an individual who exhibits behaviour consistent with the onset of schizophrenia; (iv) who is genetically predisposed to developing schizophrenia; and/or (v) having a mutated or abnormal gene associated with schizophrenia (such as the NRGI or DTNBPI gene) but who has not been diagnosed with schizophrenia.

## Patentansprüche

1. 3-Methylpent-1-yn-3-ol (Meparfynol) oder ein pharmazeutisch unbedenkliches Salz davon zur Verwendung bei der Behandlung von Schizophrenie bei einem Subjekt mit Bedarf an solch einer Behandlung.

2. Pharmazeutische Zusammensetzung, umfassend einen pharmazeutisch unbedenklichen Träger und eine wirksame Menge einer Verbindung nach Anspruch 1 als einen aktiven Bestandteil zur Verwendung bei der Behandlung von Schizophrenie.

3. Verwendung von 3-Methylpent-1-yn-3-ol (Meparfynol) oder ein pharmazeutisch unbedenkliches Salz davon bei der Herstellung eines Medikaments zur Verwendung bei der Behandlung von Schizophrenie bei einem Subjekt mit Bedarf an solch einer Behandlung.

4. Verwendung der Verbindung nach Anspruch 1 oder eines pharmazeutisch unbedenklichen Salzes davon zur Herstellung eines Medikaments zum Verlangsamen des Fortschritts von Schizophrenie bei einem Individuum, dem Schizophrenie diagnostiziert wurde; oder Verhindern oder Verzögern der Entwicklung von Schizophrenie bei einem Individuum, das gefährdet ist, Schizophrenie zu entwickeln.

5. Verwendung der Verbindung nach Anspruch 1 oder eines pharmazeutisch unbedenklichen Salzes davon zur Herstellung eines Medikaments zum Lindern von Schizophrenie bei einem Subjekt mit Bedarf an solch einer Behandlung.

6. Verwendung der Verbindung nach Anspruch 1 oder eines pharmazeutisch unbedenklichen Salzes davon zur Herstellung eines Medikaments zum Verhindern und/oder Behandeln und/oder Verzögern des Beginns und/oder der Entwicklung von Schizophrenie und/oder Verringern der Intensität oder Schwere von Schizophrenie bei einem Individuum und/oder Verbessern der Lebensqualität eines Individuums, (i) dem Schizophrenie diagnostiziert wird, (ii) das als gefährdet gilt, Schizophrenie zu entwickeln (z. B. eines Individuums, das ein oder mehrere Familienmitglieder aufweist, die Schizophrenie hatten); (iii) dem diagnostiziert wurde, dass er eine genetische Mutation aufweist, die mit Schizophrenie assoziiert ist, oder einem Individuum, das ein Verhalten aufweist, das mit dem Beginn von Schizophrenie übereinstimmt; (iv) das genetisch prädisponiert ist, Schizophrenie zu entwickeln; und/oder (v) das ein mutiertes oder anomales Gen aufweist, das mit Schizophrenie assoziiert ist (wie beispielsweise das NRGI- oder DTNBPI-Gen), dem aber keine Schizophrenie diagnostiziert wurde.

## Revendications

1. 3-méthylpent-1-yn-3-ol (méparfynol) ou un sel pharmaceutiquement acceptable de celui-ci pour l'utilisation dans le traitement de la schizophrénie chez un sujet nécessitant un tel traitement.

2. Composition pharmaceutique comprenant un vecteur pharmaceutiquement acceptable et, en tant que substance active, une quantité efficace d'un composé selon la revendication 1 pour l'utilisation dans le traitement de la schizophrénie.

3. Utilisation de 3-méthylpent-1-yn-ol (méparfynol) ou d'un sel pharmaceutiquement acceptable de celui-ci dans la fabrication d'un médicament pour l'utilisation dans le traitement de la schizophrénie chez un sujet nécessitant un tel traitement.

4. Utilisation du composé selon la revendication 1 ou d'un sel pharmaceutiquement acceptable de celui-ci pour la fabrication d'un médicament destiné à ralentir la progression de la schizophrénie chez un individu chez qui l'on a diagnostiqué une schizophrénie ; ou à prévenir ou à retarder le développement de la schizophrénie chez un individu qui présente un risque de développer une schizophrénie.

5. Utilisation du composé selon la revendication 1 ou d'un sel pharmaceutiquement acceptable de celui-ci pour la fabrication d'un médicament destiné à atténuer la schizophrénie chez un sujet nécessitant un tel traitement.

6. Utilisation du composé selon la revendication 1 ou d'un sel pharmaceutiquement acceptable de celui-ci pour la fabrication d'un médicament destiné à prévenir et/ou à traiter et/ou à retarder l'apparition et/ou le développement de la schizophrénie et/ou à réduire l'intensité ou la sévérité de la schizophrénie chez un individu et/ou à améliorer la qualité de vie d'un individu (i) chez qui l'on a diagnostiqué une schizophrénie ; (ii) qui est considéré comme étant à risque de développer une schizophrénie (par exemple, un individu dont un ou plusieurs membres de la famille ont été atteints de schizophrénie) ; (iii) chez qui l'on a diagnostiqué une mutation génétique associée à la schizophrénie ou un individu qui présente un comportement compatible avec l'apparition de la schizophrénie ; (iv) qui est génétiquement prédisposé à développer une schizophrénie ; et/ou (v) présentant un gène muté ou anormal associé à la schizophrénie (tel que le gène NRGI ou DTNBPI) mais chez qui une schizophrénie n'a pas été diagnostiquée.
